# EUROPEAN PATENT SPECIFICATION

(11) **EP 1 265 555 B1**
(45) Date of publication and mention of the grant of the patent: **23.11.2005**
(21) Application number: 01922582.0
(22) Date of filing: 22.03.2001
(51) Int. Cl.: A61F 2/28, A61F 2/46

(54) **PLUGS FOR FILLING BONY DEFECTS**
PFROPFEN ZUM FÜLLEN VON KNOCHENDEFEKTEN
BOUCHONS PERMETTANT DE COMBLER DES MALFORMATIONS OSSEUSES

(30) Priority: 22.03.2000 US 191099 P
(43) Date of publication of application: 18.12.2002
(73) Proprietor: SYNTHES (U.S.A.), Paoli, PA 19301-1222 (US)
(72) Inventor: BOYER, Michael, L., II, Paoli, PA 19301 (US); PAUL, David, C., Phoenixville, PA 19460 (US); HIGGINS, Thomas, B., Berwyn, PA 19312 (US); ANGELUCCI, Christopher, M., Schwenksville, PA 19473 (US); MESSERLI, Dominique, D., West Chester, PA 19380 (US); KOBAYASHI, Kenneth, I., Exton, PA 19341 (US)
(74) Representative: MacDougall, Donald Carmichael
(86) International application number: PCT/US2001/009270
(87) International publication number: WO 2001/070136

(56) References cited:
- EP-A- 0 505 634
- WO-A-00/07527
- WO-A-00/07528
- WO-A-00/59412
- WO-A-96/39988
- WO-A-97/25945
- WO-A-98/17209
- WO-A-98/38948
- WO-A-99/09914
- WO-A-99/38461
- DE-U- 29 913 200
- FR-A- 2 753 368
- US-A- 4 950 296
- US-A- 5 112 354
- US-A- 5 152 791
- US-A- 5 728 159
- US-A- 5 876 452
- US-A- 5 899 939

## Description

### FIELD OF THE INVENTION

The invention relates to an implant for orthopedic applications. More particularly, the invention is related to plugs for filling vacancies in bone tissue.

### BACKGROUND OF THE INVENTION

Bone grafts have become an important and accepted means for treating bone fractures and defects. In the United States alone, approximately half a million bone grafting procedures are performed annually, directed to a diverse array of medical interventions for complications such as fractures involving bone loss, injuries or other conditions necessitating immobilization by fusion (such as for the spine or joints), and other bone defects that may be present due to trauma, infection, or disease. Bone grafting involves the surgical transplantation of pieces of bone within the body, and generally is effectuated through the use of graft material acquired from a human source. This is primarily due to the limited applicability of xenografts, transplants from another species.

Orthopedic autografts or autogenous grafts involve source bone acquired from the same individual that will receive the transplantation. Thus, this type of transplant moves bony material from one location in a body to another location in the same body, and has the advantage of producing minimal immunological complications. It is not always possible or even desirable to use an autograft. The acquisition of bone material from the body of a patient typically requires a separate operation from the implantation procedure. Furthermore, the removal of material, oftentimes involving the use of healthy material from the pelvic area or ribs, has the tendency to result in additional patient discomfort during rehabilitation, particularly at the location of the material removal. Grafts formed from synthetic material have also been developed, but the difficulty in mimicking the properties of bone limits the efficacy of these implants.

As a result of the challenges posed by auto grafts and synthetic grafts, many orthopedic procedures alternatively involve the use of allografts, which are bone grafts from other human sources (normally cadavers). The bone grafts, for example, are placed in a host bone and serve as the substructure for supporting new bone tissue growth from the host bone. The grafts are sculpted to assume a shape that is appropriate for insertion at the fracture or defect area, and often require fixation to that area as by screws or pins. Due to the availability of allograft source material, and the widespread acceptance of this material in the medical community, the use of allograft tissues is certain to expand in the field of musculoskeletal surgery.

Notably, the various bones of the body such as the femur (thigh), tibia and fibula (leg), humerus (upper arm), radius and ulna (lower arm) have geometries that vary considerably. In addition, the lengths of these bones vary; for example, in an adult the lengths may vary from 47 centimeters (femur) to 26 centimeters (radius). Furthermore, the shape of the cross section of each type of bone varies considerably, as does the shape of any given bone over its length. While a femur has a generally rounded outer shape, a tibia has a generally triangular outer shape. Also, the wall thickness varies in different areas of the cross-section of each bone. Thus, the use of any given bone to produce an implant component may be a function of the bone's dimensions and geometry. Machining of bones, however, may permit the production of implant components with standardized dimensions.

As a collagen-rich and mineralized tissue, bone is composed of about forty percent organic material (mainly collagen), with the remainder being inorganic material (mainly a near-hydroxyapatite composition resembling 3Ca₃(PO₄)₂· Ca(OH)₂). Structurally, the collagen assumes a fibril formation, with hydroxyapatite crystals disposed along the length of the fibril, and the individual fibrils are disposed parallel to each other forming fibers. Depending on the type of bone, the fibrils are either interwoven, or arranged in lamellae that are disposed perpendicular to each other.

There is little doubt that bone tissues have a complex design, and there are substantial variations in the properties of bone tissues with respect to the type of bone (*i*.*e*., leg, arm, vertebra) as well as the overall structure of each type. For example, when tested in the longitudinal direction, leg and arm bones have a modulus of elasticity of about 17 to 19 GPa, while vertebra tissue has a modulus of elasticity of less than 1 GPa. The tensile strength of leg and arm bones varies between about 120 MPa and about 150 MPa, while vertebra have a tensile strength of less than 4 MPa. Notably, the compressive strength of bone varies, with the femur and humerus each having a maximum compressive strength of about 167 MPa and 132 MPa respectively. Again, the vertebra have a far lower compressive strength of no more than about 10 MPa.

With respect to the overall structure of a given bone, the mechanical properties vary throughout the bone. For example, a long bone (leg bone) such as the femur has both compact bone and spongy bone. Cortical bone, the compact and dense bone that surrounds the marrow cavity, is generally solid and thus carries the majority of the load in major bones. Cancellous bone, the spongy inner bone, is generally porous and ductile, and when compared to cortical bone is only about one-third to one-quarter as dense, one-tenth to one-twentieth as stiff, but five times as ductile. While cancellous bone has a tensile strength of about 10-20 MPa and a density of about 0.7, cortical bone has a tensile strength of about 100-200 MPa and a density of about 2. Additionally, the strain to failure of cancellous bone is about 5-7%, while cortical bone can only withstand 1-3% strain before failure. It should also be noted that these mechanical characteristics may degrade as a result of numerous factors such as any chemical treatment applied to the bone material, and the manner of storage after removal but prior to implantation (i. e. drying of the bone).

Notably, implants of cancellous bone incorporate more readily with the surrounding host bone, due to the superior osteoconductive nature of cancellous bone as compared to cortical bone. Furthermore, cancellous bone from different regions of the body is known to have a range of porosities. For example, cancellous bone in the iliac crest has a different porosity from cancellous bone in a femoral head. Thus, the design of an implant using cancellous bone may be tailored to specifically incorporate material of a desired porosity.

It is essential to recognize the distinctions in the types and properties of bones when considering the design of implants. Surgeons often work with bones using similar tools as would be found in carpentry, adapted for use in the operating room environment. This suggests that bones have some properties which are similar to some types of wood, for example ease in sawing and drilling. Notably, however, are many differences from wood such as the abrasive nature of hydroxyapatite and the poor response to local heating during machining of a bone. The combination of tensile and compressive strengths found in bone, resulting from the properties of the collagen and hydroxyapatite, is thus more aptly compared to the tensile and compressive strengths found in reinforced concrete, due to steel and cement. Furthermore, while wood is readily available in considerable quantity, bone material is an extremely limited resource that must be used in an extremely efficient manner.

Various types of bone grafts are known. For example, as disclosed in U.S. Patent No. 5,989,289 to Coates et al., a spinal spacer includes a body formed of a bone composition such as cortical bone. The spacer has walls that define a chamber that is sized to receive an osteogenic composition to facilitate bone growth.

U.S. Patent No. 5,899,939 to Boyce et al. discloses a bone-derived implant for load-supporting applications. The implant has one or more layers of fully mineralized or partially demineralized cortical bone and, optionally, one or more layers of some other material. The layers constituting the implant are assembled into a unitary structure, as by joining layers to each other in edge-to-edge fashion in a manner analogous to planking.

WO 97/25945 relates to a diathysial cortical dowel provided by obtaining a plug from a shaft of various long bones. The dowel has an intra-medullary canal which can be packed with any of a variety of osteogenic materials.

With a rapidly increasing demand in the medical profession for devices incorporating bone material, the tremendous need for the tissue material itself, particularly allograft tissue material, presents a considerable challenge to the industry that supplies the material. Due to the size and shape of the bones from which the material is harvested, and the dimensional limitations of any particular type of bone in terms of naturally occurring length and thickness (i.e. cortical or cancellous), there is a need for a means by which individual bone fragments can be combined to form larger, integral implants that are more suitable for use in areas of larger fractures or defects. For example, the size of cortical bone fragments needed to repair a fracture or defect site is often not available in a thick enough form. While multiple fragments may together meet the size and shape requirements, several prominent concerns have placed a practical limitation on the implementation of this concept. There is considerable uncertainty regarding the structural integrity provided by fragments positioned adjacent to one another without bonding or other means of securing the fragments to each other. Moreover, there is concern over the possibility that a fragment may slip out of position, resulting in migration of the garment and possible further damage in or near the area of implantation.

In addition, due to the geometry of bones such as the femur and tibia, all portions of the bones are not readily usable as a result of size limitations. Thus, prior art implants, specifically allografts, are produced with an inefficient use of source bones.

There is a need for new, fundamental approaches to working with and processing tissues, in particular allograft material, especially with regard to machining, mating, and assembling bone fragments. Specifically, there is a need for an implant that allows more efficient use of source material. More specifically, there is a need for an implant that is an integrated implant comprising two or more bone fragments that are interlocked to form a mechanically effective, strong unit.

### SUMMARY OF THE INVENTION

According to a first aspect of the present invention there is provided a plug for filling a vacant region in anatomical bone, comprising:
a body with a top end, a bottom end, and an outer surface disposed between the top and bottom ends; and
a first cap comprising a locking portion mechanically lockable to an end of the body and configured and dimensioned to be rotatably received in the body,
wherein the locking portion extends substantially across a face of the first cap, and
wherein the body and first cap are substantially formed from bone selected from the group consisting of cortical and cancellous bone.

The present invention is related to a plug for filling a vacancy in bone tissue. The plug includes a body and at least one end cap coupled together. The body may be a sleeve with a top end, a bottom end, an inner surface and an outer surface. An insert configured and dimensioned to be received in the sleeve may also be provided. The insert may be formed of cancellous bone which may have a fluid concentrated therein. The insert may be subjected to mechanical pressure to concentrate the fluid, which may be applied by aspiration. The fluid also may be concentrated by soaking.

In some embodiments, the insert is secured to at least one of the sleeve and end cap with at least one fastener that is selected from a screw, key, pin, peg, rivet, cotter, nail, spike, bolt, stud, staple, boss, clamp, clip, dowel, stake, hook, anchor, tie, band, crimp, and wedge. At least one of the sleeve, end cap, insert, and fastener may be formed from partially demineralized or demineralized bone, and at least two of the sleeve, end cap, insert, and fastener may be bonded together with a bonding agent. At least one of the sleeve, end cap, insert, and fastener may be at least partially dehydrated to loosely fit within a surrounding mating surface. At least one of the sleeve, end cap, and insert may include alignment indicia. In some embodiments, the sleeve may be packed with at least one of bone chips, bone particulate, bone fibers, bone growth materials, hydroxyapatite, metal, resorbable material, polymer, ceramic, and bone cement.

In some embodiments, the sleeve also may include at least one through-hole extending from the inner surface to the outer surface, and the sleeve may be cylindrical. In addition, the sleeve may include at least one depression extending from the outer surface toward the inner surface. Furthermore, the sleeve may include a plurality of fingers formed integrally therewith.

The body and the at least one end cap of the plug may be formed from bone, with the sleeve and end cap being formed of cortical bone.

The present invention also is related to a plug for filling a vacant region in anatomical bone. The plug includes a body with a top end, a bottom end, and an outer surface disposed between the top and bottom ends. In addition, the plug includes at least one cap disposed at an end of the body, with the body and at least one cap being formed from bone. In some embodiments, the body and cap are integrally formed. The body and cap may be formed from a section of a long bone taken transverse to a long axis of the long bone, with the body including a through-hole formed by a canal in the long bone. In another embodiment, the body may be formed of cancellous bone and the cap may be formed of cortical bone.

The present invention further relates to a method for filling a vacancy in bone, including: inserting a filler into a portion of the vacancy, the filler comprising at least one of bone chips, bone particulate, bone fibers, bone-growth materials, hydroxyapatite, metal, resorbable material, polymer, ceramic, and bone cement; and fitting a cap to the vacancy to seal the filler therein.

### BRIEF DESCRIPTION OF THE DRAWINGS

Preferred features of the present invention are disclosed in the accompanying drawings, wherein similar reference characters denote similar elements throughout the several views, and wherein:
FIGS. 1A and 1B show perspective views of embodiments of plugs according to the present invention;
FIGS. 1C to 1G show exploded, perspective views of exemplary plugs;
FIG. 1H shows a perspective view of another plug;
FIGS. 1I to 1J show exploded, perspective views of further plugs;
FIGS. 2A to 2H show perspective views of embodiments of caps according to the present invention;
FIG. 3A shows a side view of a sleeve for a plug according to the present invention;
FIG. 3B shows a cross-section of the sleeve of FIG. 3A through line IIIB-IIIB;
FIG. 3C shows a cross-section of the sleeve of FIG. 3A through line IIIC-IIIC;
FIG. 3D shows a perspective view of a cap according to the present invention;
FIG. 3E shows a bottom view of the cap of FIG. 3D;
FIG. 3F shows a side view of the cap of FIG. 3D;
FIG. 3G shows a perspective view of the sleeve of FIG. 3A;
FIGS. 3H to 3J show the installation of the cap of FIG. 3D on the sleeve of FIG. 3G;
FIGS. 3K and 3L show perspective views of fillers according to the present invention;
FIG. 3M shows a side view of a plug according to the present invention with a filler formed from a femoral head;
FIG. 3N shows another embodiment of a sleeve for a plug according to the present invention with threading;
FIGS. 4A to 4C show a top view, side view, and another side view, respectively, of an additional embodiment of a cap according to the present invention;
FIG. 4D shows a side view of the cap of FIG. 4A installed in a sleeve of a plug according to the present invention;
FIG. 4E shows a cross-section of the cap of FIG. 4A through line IVE-IVE;
FIG. 4F shows a bottom view of a cap with a locking portion having serrated edges according to the present invention;
FIG. 4G shows a perspective view of a sleeve with recesses on the outer surface for a plug formed according to the present invention;
FIG. 5A shows a side view of an additional embodiment of a sleeve for a plug with fingers according to the present invention;
FIG. 5B shows a cross-section of the sleeve of FIG. 5A through line VB-VB;
FIG. 5C shows a cross-section of the sleeve of FIG. 5A through line VC-VC;
FIGS. 5D and 5E show top and side views, respectively, of an additional embodiment of a cap for a plug according to the present invention;
FIG. 6A shows a perspective view of an additional embodiment of a plug according to the present invention;
FIG. 6B shows an exploded, perspective view of another embodiment of a plug according to the present invention;
FIGS. 7A and 7B show the forming of a cancellous plug between a pair of dies according to the present invention;
FIG. 8A shows a side view of another plug;
FIG. 8B shows a side view of the plug of FIG. 8A;
FIG. 9A shows a side view of another plug;
FIG. 9B shows a side view of the plug of FIG. 9A;
FIG. 10A shows a side view of another plug;
FIG. 10B shows a side view of the plug of FIG. 10A;
FIG. 11A shows a side view of another plug;
FIG. 11B shows a side view of the plug of FIG. 11A;
FIG. 12 shows a side view of yet another plug;
FIG. 13 shows a side view in cross-section of another plug;
FIG. 14A shows a side view in cross-section of another plug;
FIG. 14B shows a side view of the plug of FIG. 14A;
FIG. 15A shows a side view in cross-section of another plug;
FIG. 15B shows a side view of the plug of FIG. 15A;
FIGS. 16 to 25 show side views in cross-section of additional embodiments of plugs;
FIGS. 26 and 27 show exploded perspective views of additional plugs
FIGS. 28 and 29 show perspective views of insertable components for use with plugs;
FIG. 30A shows a side view in cross-section of another plug;
FIG. 30B shows a perspective view of the sleeve of FIG. 30A;
FIGS. 30C and 30D show a side view and bottom view, respectively, of the end cap of FIG. 30A;
FIGS. 31A and 31B show the formation of a plug from a cross-section of a bone taken transverse to the long axis of a bone; and
FIGS. 32A and 32B show partial cross-sections of a long bone and a vertebral body, respectively, with a bone plug inserted therein.

### DETAILED DESCRIPTION OF THE PREFERRED EMBODIMENTS

Any of a wide variety of different implant structures, particularly allograft, autograft, and/or xenograft implant structures, can be prepared according to the teachings of the present invention. While a representative selection of implant structures are described and depicted herein, additional disclosure is found in U.S. Provisional Application No. 60/191,099 filed March 22, 2000.

As used in the description of the present invention, the words fitting, interfitting, mating, locking, interlocking, meshing, and interlacing are all used generically to describe the joining of bone sections or pieces together. Thus, these words are not limited to the use of any particular manner of joining. Thus, for example, the press-fitting of one bone section within a cavity formed in another bone section may be described using any of the above-mentioned terms. In addition, although various preferred mechanical fastening approaches are described, the present invention allows the use of any mechanical device for joining two or more separate parts of an article or structure. Such mechanical devices include, but are not limited to the following: screws, keys, pins, pegs, rivets, cotters, nails, spikes, bolts, studs, staples, bosses, clamps, clips, dowels, stakes, hooks, anchors, ties, bands, and crimps. Also, bonding agents or other chemical means for joining two separate parts may be employed alone or in combination with the mechanical devices. Thus, as appropriate, the means disclosed herein for fixing bone sections to each other may be substituted, as with the above-mentioned mechanical devices, bonding devices, or chemical means. Furthermore, although particular types of joints are disclosed, the present invention is directed to the creation of implants that may be joined using other joints.

While the present invention is preferably directed to the creation of implants from allograft material, the present invention may also be applied to implants that utilize other materials, including but not limited to the following: xenograft, autograft, metals, alloys, ceramics, polymers, composites, and encapsulated fluids or gels. Furthermore, the implants described herein may be formed of materials with varying levels of porosity, such as by combined bone sections from different bones or different types of tissue having varying levels of porosity.

Also, the implants described herein may be formed of bone materials with varying mineral content. For example, cancellous or cortical bone may be provided in natural, partially demineralized, or demineralized states. Demineralization is typically achieved with a variety of chemical processing techniques, including the use of an acid such as hydrochloric acid, chelating agents, electrolysis or other treatments. The demineralization treatment removes the minerals contained in the natural bone, leaving collagen fibers with bone growth factors including bone morphogenic protein (BMP). Variation in the mechanical properties of bone sections is obtainable through demineralization. Advantageously, use of a demineralizing agent on natural bone transforms the properties of the bone from a stiff structure to a relatively pliable structure when hydrated. Some portions of interfitting bone components may be demineralized or partially demineralized in order to achieve improved interfitting. For example, a tissue form may include two bone components having portions that are coupled together with an interference fit. The interference fit may be enhanced if the surface region of one or more of the components is demineralized or partially demineralized so that it is pliable and exhibits some elasticity and/or malleability when hydrated.

In addition, while many of the embodiments described herein show bone components disposed at right angles, or joints formed with right angles, angles that are greater or less than ninety degrees may alternatively be used in implants of the present development.

The components that are used to create implants of the present invention may all be formed from cortical bone, all from cancellous bone, or a combination of components formed from cortical and cancellous bone. The interfitting of the components may be achieved through a variety of means, including but not limited to the following: pinning, bonding with a suitable bone bonding agent or chemical means, press fitting, threadably engaging (as by helically screwing one component into another), inserting a tapered component into a component with a matching inner surface, twist-locking, or other interlocking means such as will be described in other embodiments. While the present development preferably allows the creation of implants from all bone material, it is also anticipated that one or more components used to create the implants may be formed of non-bone material such as a synthetic or other material. Thus, while the implants disclosed herein are typically described as being formed primarily from bone, the implants alternatively may be formed in whole or in part from other materials such as hydroxyapatite, metal, resorbable material, polymer, and ceramic, and may additionally incorporate bone chips, bone particulate, bone fibers, bone growth materials, and bone cement. Also, while solid, cylindrical sleeve-like structures are described herein, the sleeves optionally may include perforations extending from outer to inner surfaces, or recesses formed in outer surfaces that do not extend through inner surfaces. Geometries such as circular depressions, dimples formed from a spherical geometry, diamond shapes, or rectangular shapes may be used.

Bones suitable for forming implants of the present invention include a radius, humerus, tibia, femur, fibula, or ulna, although other bones may be used.

The moisture content of the bone sections also may be varied to advantageously permit improved interlocking. Bone components initially may be provided with moisture content as follows: (1) bone in the natural state fresh out of the donor without freezing, (2) bone in the frozen state, typically at -40°C, with moisture content intact, (3) bone with moisture removed such as freeze-dried bone, and (4) bone in the hydrated state, such as when submersed in water. Using the expansion and contraction properties that can be obtained during heating and cooling of the bone material, and the concomitant resorption of moisture along with swelling for some bone material, permits an alternate approach to achieving a tight press-fit.

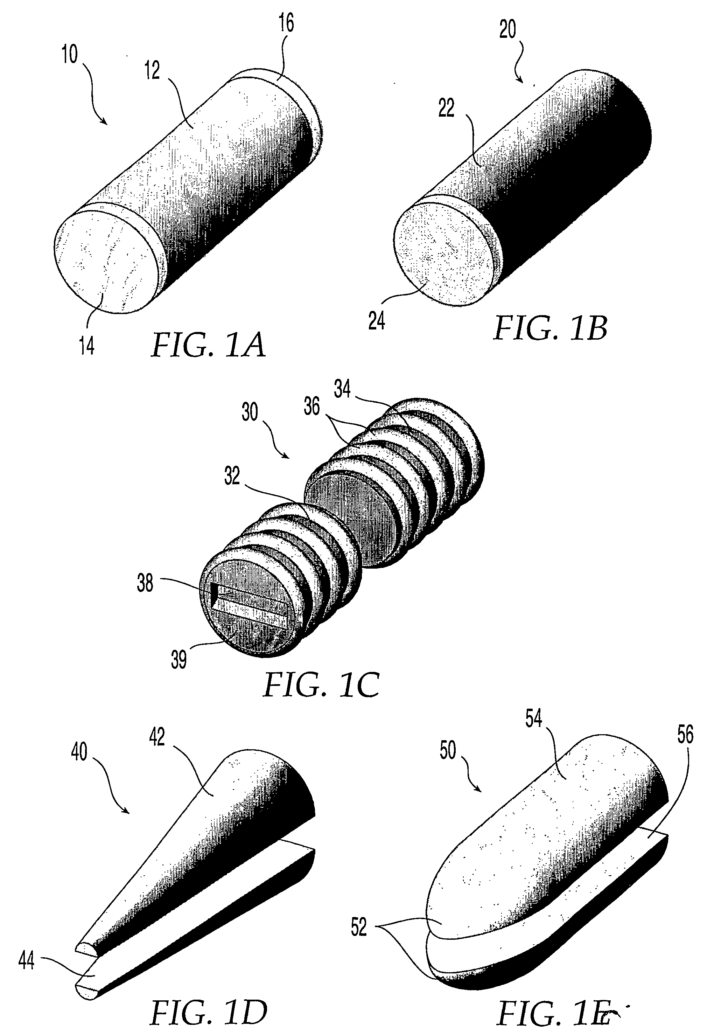

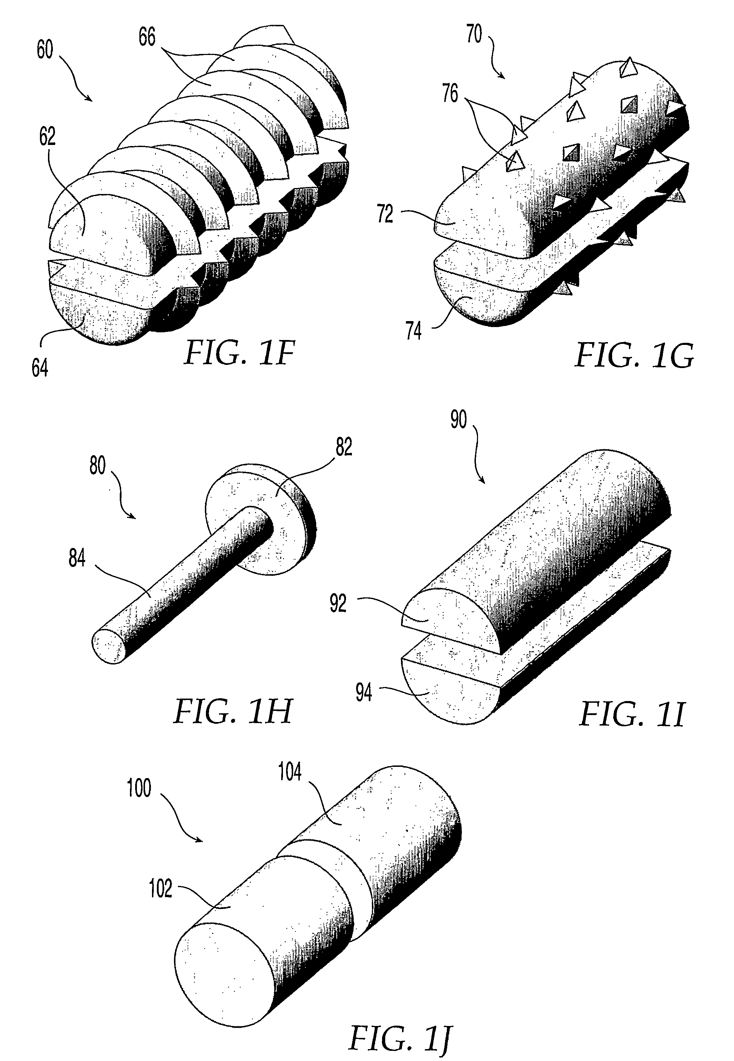

Turning to FIGS. 1A to 1J, tissue forms are shown in the shape of plugs suitable for implantation. Plugs may be created using a core drill or trephine system, or may be machined or otherwise formed from bone. For example, using a trephine system, essentially a dowel cutter for use with tissues, a cylindrical shaped plug may be cut *in vivo* from a tissue, such as a vertebral body or tissue from another location such as the hip. This material may be used as an autograft, which may serve as a plug to fill an anatomical defect or instead may serve as a strut to be inserted into another implant formed of bone to provide additional strength. A similarly sized plug of allograft material may be used to fill the hole created by the removal of autograft material. The plugs contemplated by the present invention, however, may be made of allograft or xenograft bone material as well, or combinations of autograft, allograft, and xenograft bone material. The plugs may also be formed from cancellous bone, cortical bone, or combinations thereof and the choice of such materials may be based on the materials properties obtainable from a given type of bone. For example, cancellous bone is available in a range of porosities based on the location in the body from which the bone is harvested. Extremely porous cancellous bone may be harvested from various areas such as the iliac crest, while less porous bone may be harvested from areas such as a tibial condyle. Thus, the materials properties - particularly the porosity - of the plugs may be selected to meet the needs of a given application. In addition, the plugs of the present invention may be formed either partially or completely using non-bone materials such as metals, alloys, ceramics, polymers, composites, and encapsulated fluids or gels.

Turning to the various embodiments of plugs according to the present invention, it should be noted that the plugs discloses herein may be of unitary construction, or may be formed from multiple pieces that are interfitted together. Thus, while the figures may show cross-sections of plugs or plugs formed with multiple pieces to produce a particular structure, it should be noted that the structures instead may be one-piece. In addition, although the plugs are generally described herein as being formed from bone or at least in part from bone, the plugs may additionally be formed completely from non-bone materials such as metals or ceramics. Also, while dimensions are indicated herein for plugs, it should be noted that much smaller plugs as well as much larger plugs also are contemplated following the general structures disclosed herein.

As shown in FIG. 1A, a cylindrical plug 10 according to the present invention includes a central region 12 and end caps 14, 16. Preferably, central region 12 is formed of cancellous bone material, while caps 14, 16 are formed of cortical bone material. Cortical bone material is placed at the ends of plug 10 due to its superior mechanical strength and lower porosity as compared to cancellous bone material. In alternate embodiments, central region 12 and end caps 14, 16 may be formed from other types of bone and or non-bone materials to suit a particular need.

Other plugs are shown in FIGS. 1B to 1J. Plug 20 includes a body 22 and single end cap 24. Plug 30 is formed of two sections of bone 32, 34 that are interfitted together. Ribbing 36 may be provided along the length of plug 20, and a keyhole 38 may be provided at a head 39. Keyhole 38 preferably is sized to receive a screwdriver, or other suitable device, to facilitate the installation of plug 30 in an anatomical region. Alternate forms of keyhole 38 include recesses shaped in the form of a cruciform, hex, star, recess, or clover. In another embodiment, a tapered, conical shaped plug 40 may be formed from bone material. Plug 40 may be formed from a single piece of bone, or it may be formed of two or more pieces of bone that are interfitted together as shown for example with sections 42, 44. Similarly, a bullet shaped plug 50 with a blunt tip 52 may be formed from bone sections 54, 56.

A plug 60 as shown in FIG. 1F is formed of bone sections 62, 64 that are interfitted together, and further includes threading 66 that is machined on the plug. A plug 70, also formed for example from two bone sections 72, 74 includes migration-resistance protrusions such as spurs or teeth 76 disposed around the circumference of the plug. Alternatively, a plug 70 could be formed from a single piece of bone or other material such as metal or ceramic.

A plug 80 with an oversized head 82 and a shaft 84 is shown in FIG. 1H. As previously discussed with respect to embodiments with end caps, head 82 may be formed of cortical bone while shaft 84 may be formed of cancellous bone. Although plug 80 may be machined from one piece of bone, head 82 and shaft 84 may be separate bone pieces that are interfitted together. Similarly, plug 80 may incorporate non-bone material. Other designs of plugs include a plug 90 formed from two bone sections 92, 94 that are interfitted together along a longitudinal axis extending through the free ends of the sections, and plug 100 formed from bone sections 102, 104 that are interfitted together at one free end of each section. Alternate embodiments contemplated by the present invention include plugs formed of non-symmetrical bone sections, plugs formed from one piece of bone, and plugs formed from more than two pieces of bone with the sections joined together using joints, fasteners, or other techniques described above. In addition, the plugs may be formed using metal, resorbable material, plastic, or ceramic, as well as the various types of bone with various degrees of porosity as previously described. Plug may include sections of cortical bone, cancellous bone, metals ceramics, or other materials.

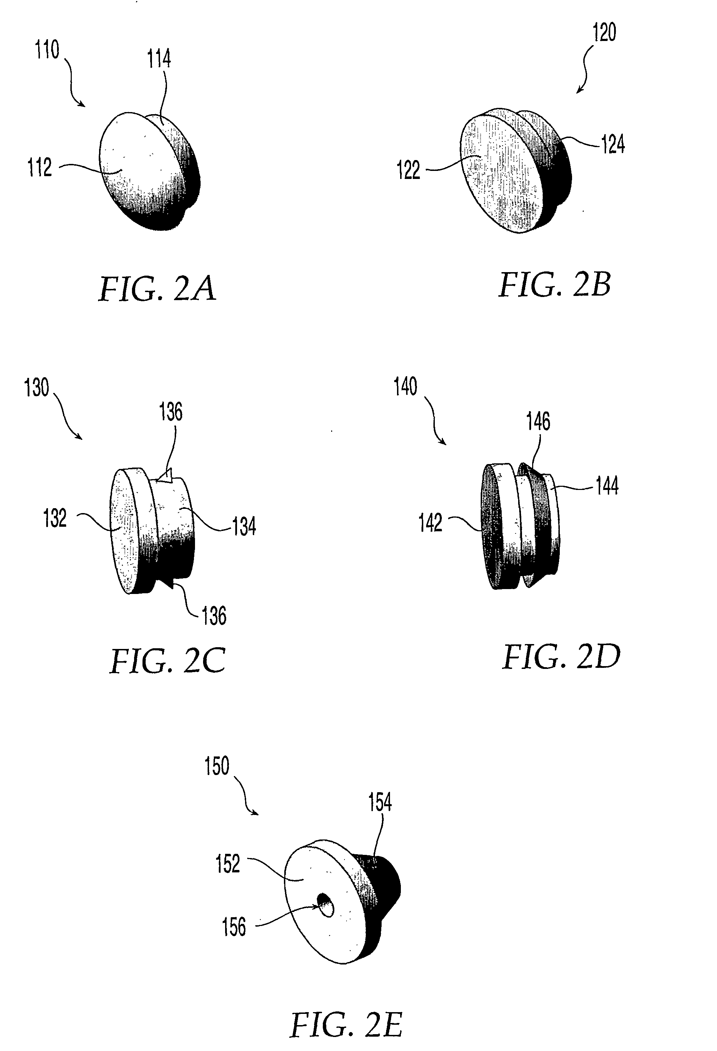

In some circumstances it is desirable not to fill an entire defect or vacant region in a bone with a single plug. For example, other material such as bone chips, slurries of bone particulate, bone fibers, bone-growth inducing substances, bone cement, or polymers may be inserted into the defect or vacancy, and sealed therein using a cap. Various forms of caps suitable for this purpose are shown in FIGS. 2A-2H. The caps may be created using allograft tissue. In alternate embodiments, the caps may be formed using metal, resorbable material, plastic, ceramic, autograft, or xenograft. The cap 110 shown in FIG. 2A includes a rounded head 112 and a body 114 which may be press-fit into a vacancy in bone, while cap 120 includes a flat head 122 and a body 124. Cap 130, shown in FIG. 2C, includes a cap 132, body 134, and migration-resistant protrusions 136, while cap 140 includes a head 142, body 144, and flange area 146 that functions similar to circumferentially distributed teeth. Another cap of the present invention, cap 150, includes a head 152 and a tapered body 154. A through-hole 156 may also be provided, for example, to permit a needle to gain access to the vacancy sealed by the cap. Such a hole may permit delivery of substances after the cap has been installed, or permit expansion of the cap as by inserting a component therein.

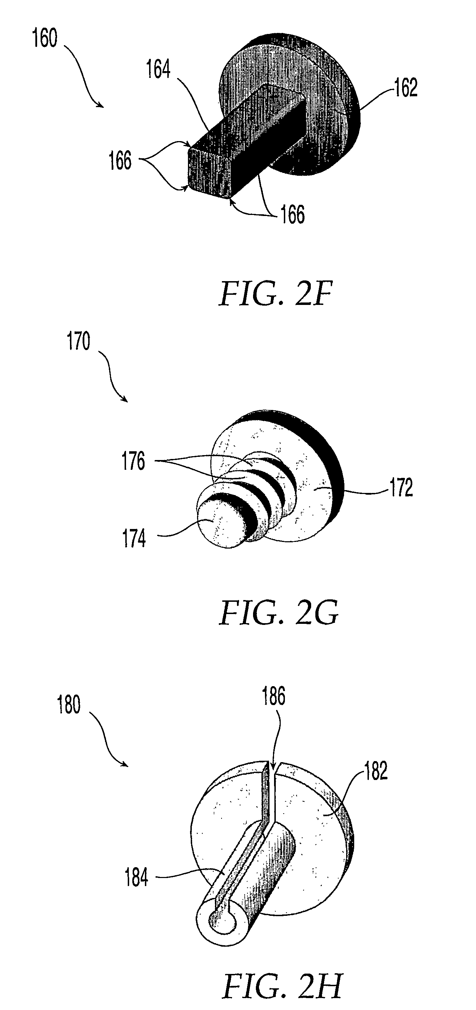

Cap 160, shown in FIG. 2F, includes a flat, circular head 162 and a generally rectangular body 164. Notably, the faces of body 164 meet to create four longitudinal radiused portions 166. When cap 160 is inserted in a suitably sized vacancy, the four radiused portions 166 provide four regions of contact with the walls of the vacancy. Such a construction facilitates press-fitting in the vacancy; a press-fit of a cap body that is closely shaped to conform to the walls of a defect may be difficult to achieve due to the tightness inherent in the fit itself. A less tight fit, as provided for example by body 164, may permit a press-fit to be achieved with less difficulty. While a press-fit with four radiused portions 166 of contact has been described, it is also contemplated that press-fits with other amounts of contact may be used.

Several additional caps are shown in FIGS. 2G and 2H. Cap 170 includes a head 172 and a body 174 with threading 176. Cap 180 includes a head 182 and a body 184. A slit 186 extends about halfway through head 182 and body 184. Slit 186 allows cap 180 to be compressed when inserted in a vacancy; after insertion, the cap may re-expand to more closely fit against the walls of the vacancy. Also, cap 180 may be inserted into a vacancy and expanded using a wedge or pin that is subsequently inserted into slit 186.

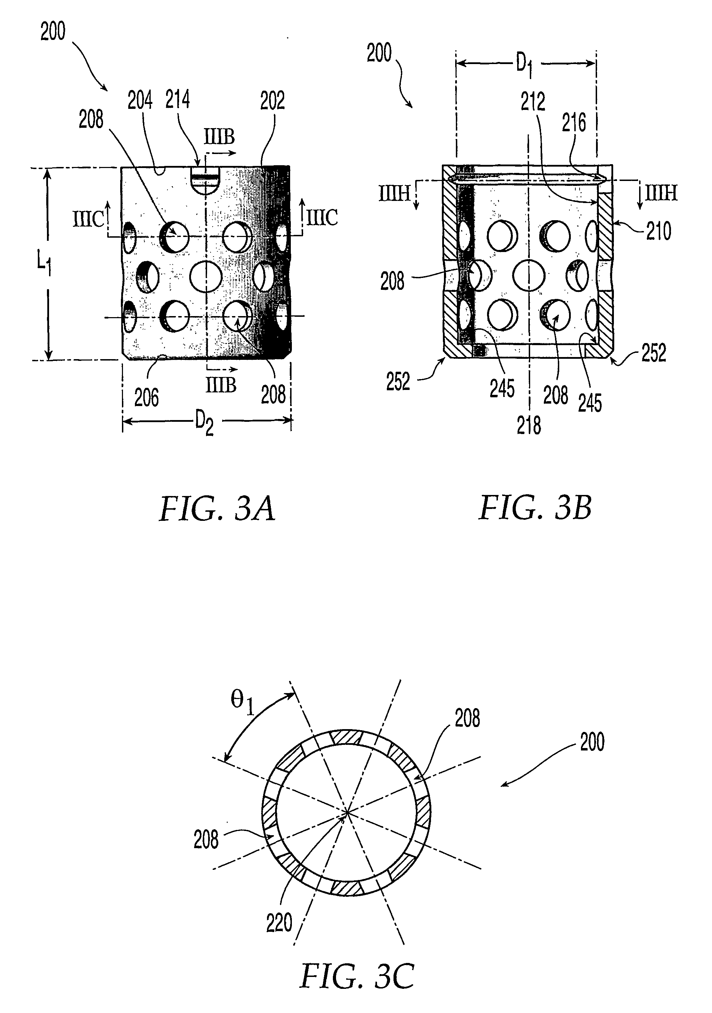

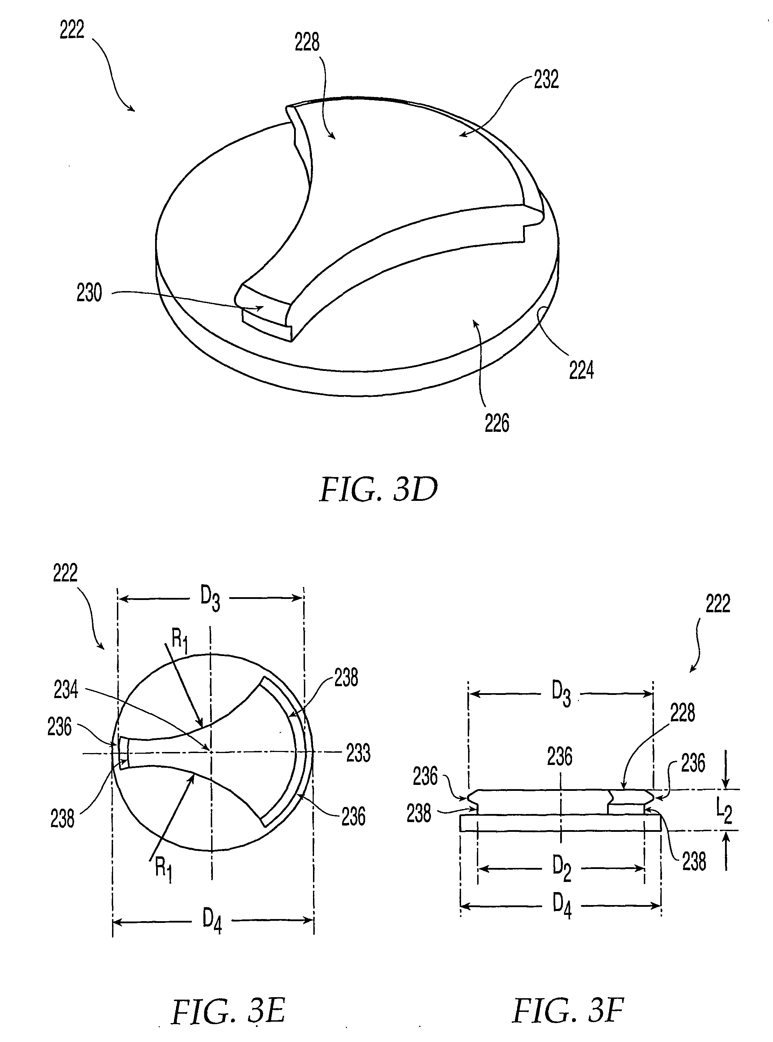

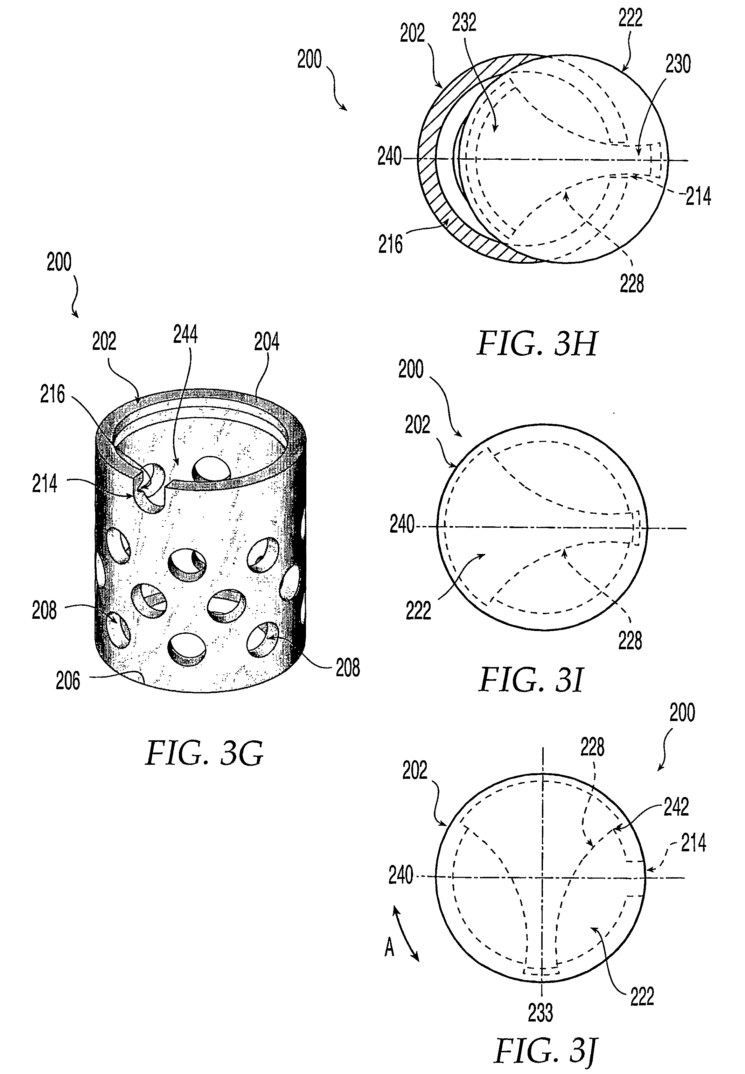

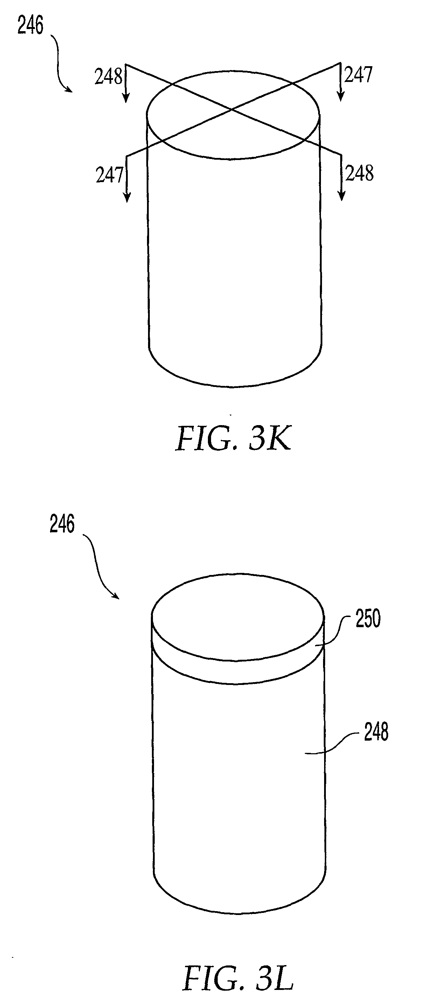

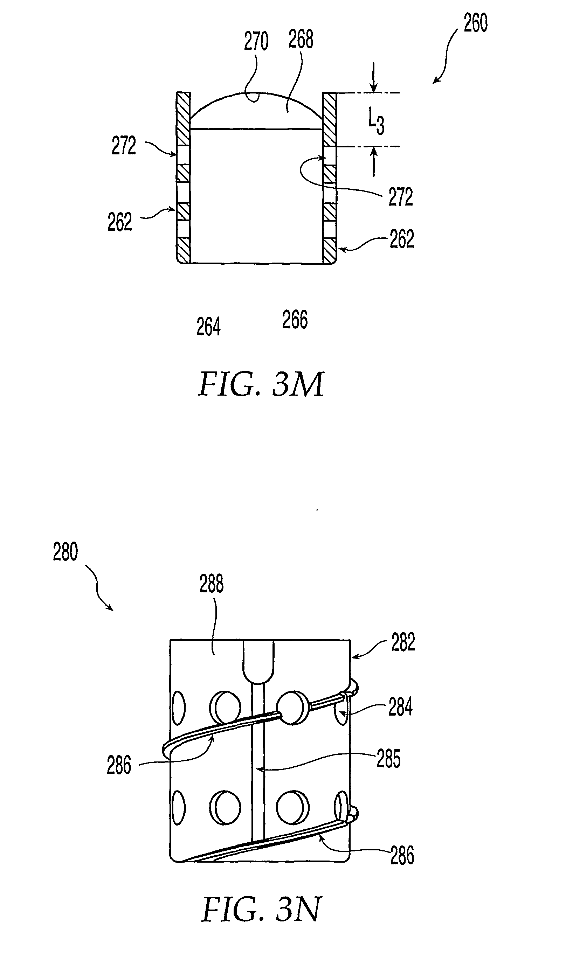

Referring initially to FIGS. 3A to 3C, a preferred embodiment of a plug according to the present invention is shown. Plug 200 includes a sleeve 202 with open, free ends 204, 206. Holes 208 extend from outer wall 210 to inner wall 212. Preferably, holes 208 are arranged in rows centered about parallel planes with respect to each other, around the circumference of plug 200. A slot 214 is disposed at a free end 204 and a circumferential groove 216 is cut into inner surface 212 proximate free end 204. A central axis 218 is disposed longitudinally about the center of plug 200, and preferably inner and outer diameters D₁, D₂, respectively, of plug 200 are generally constant, but may be tapered. In the preferred embodiment, holes 208 are offset by a generally constant angle θ₁ with respect to each other, as measured from center 220 defined along axis 218. Preferably, angle θ₁ is between about 35° and about 55° and more preferably about 45°. In a preferred embodiment, plug 200 has an inner diameter D₁ of between about 1.2 cm and 1.4 cm, an outer diameter D₂ of between about 1.5 cm and 1.7 cm, and a length L₁ of between about 1.6 cm and 2.0 cm. More preferably, inner diameter D₁, outer diameter D₂, and length L₁ are about 1.3 cm, 1.6 cm, and 1.9 cm, respectively. Holes 208 are preferably circular and have a diameter of between about 0.2 cm and about 0.5 cm, and more preferably about 0.35 cm. In alternate embodiments, holes 208 may be in other shapes such as elliptical or oblong geometries; the number of holes 208 as well as the alignment of holes 208 with respect to each other may be varied.

As shown in FIGS. 3D to 3F, a cap 222 suitable for coupling to sleeve 202 includes a top surface 224 and a bottom surface 226. A locking portion 228 preferably is integrally formed on bottom surface 226, and includes narrow and wide portions 230, 232, respectively, which are symmetrical about central line 233. The outer edges of narrow and wide portions 230, 232 are preferably disposed along the circumference of a circle centered about point 234 along central axis 236 of cap 222, with the diameter of the circle being smaller than that of outer diameter D₂ of sleeve 202. In particular, as shown in FIG. 3F, locking portion 228 of cap 222 includes a lip 236 and a wall 238. Preferably, outer diameter D₄ of cap 222 is about the same as outer diameter D₂ of sleeve 202, lip 236 of cap 222 has an outer diameter D₃ sized to fit and turn within groove 216 in sleeve 202, while wall 238 has an outer diameter D₂ sized to fit and turn proximate inner surface 212 of sleeve 202. In addition, locking portion 228 preferably tapers from wide portion 232 to narrow portion 230 along a radius of curvature R₁ of between about 0.5 cm and 1.1 cm, and more preferably 0.8 cm. Preferably, the overall thickness L₂ of cap 222 is between about 0.2 cm and about 0.5 cm.

Referring to FIGS. 3G to 3J, free end 204 of sleeve 202 preferably is closed using a cap 222. Cap 222 is coupled to sleeve 202 by first placing cap 222 with wide portion 232 of locking portion 228 centrally disposed within free end 204 of sleeve 202 and narrow portion 230 of locking portion 228 disposed in slot 214 of sleeve 202. In this position, central line 233 of cap 222 is aligned with central line 240 extending along a diameter of sleeve 202, centrally through slot 214. With lip 236 of cap 222 disposed in groove 216 of sleeve 202, cap 222 is securely coupled to sleeve 202 by rotating cap 222 in the direction of arrow A, such that lines 233, 240 of cap 222 and sleeve 202, respectively, are no longer colinear. In some embodiments, groove 216 is interrupted to create a stop (not shown), as for example located at region 242, thereby preventing additional rotation of cap 222.

With a cap 222 installed on sleeve 202, plug 200 includes a chamber 244 with one end open. This chamber 244 may be packed with such materials as bone chips, slurries of bone particulate, bone fibers, bone-growth inducing substances, hydroxyapatite, polymers such as polymethylinethacrylate, ceramics, bone cement, or other materials. In a preferred embodiment, sleeve 202 and cap 222 are formed of cortical bone, and chamber 244 is packed with a cylinder 246 that is preferably formed of cancellous bone and inserted prior to installation of cap 222. Alternatively, other materials such as porous ceramics may be used. Holes 208 facilitate incorporation of plug 200 into surrounding bone tissue, by permitting ingrowth as well as access to materials retained in chamber 244. If a solid cylinder 246 having a diameter of about D₁ is inserted into chamber 244, the cylinder may be prevented from removal through free end 206 by a circumferential lip 245. Thus, an end cap is not required at free end 206 to retain cylinder 246. The cortical bone may be obtained from the thin cortical bone proximate the ends of long bones, such as the humerus or tibia, or other regions. A sleeve 202 and cap 222 formed of cortical bone provide structural integrity to plug 200, and advantageously the cortical bone is readily machinable to repeatable dimensions and desirable tolerances. In an alternate embodiment, cylinder 246 may be formed of a main cancellous body portion 248 and a cortical end cap 250, with cortical end cap 250 disposed in a free end of sleeve 202 such that both free ends are closed by cortical bone for added structural integrity. In another alternate embodiment, cylinder 246 may be used as a plug. Although cylinder 246 is shown as a one-piece component, cylinder 246 alternately may be formed of two or more portions, for example, by sectioning cylinder 246 along lines 247, 248. A sectioned cylinder 246 may loosely fit in a sleeve, with the sectioning permitting expansion of the cylinder portions or contraction of the sleeve without creating significant stress on the components.

When plug 200 is inserted into an anatomical vacancy, preferably open free end 206 is inserted first such that cortical cap 222 faces outward. Other materials may be used to form plug 200, such as metals or ceramics. To facilitate insertion of plug 200, the circumferential edge 252 proximate free end 206 preferably is chamfered to assist in guiding plug 200 into the vacancy.

In an alternate embodiment, a plug 260 includes a cylindrical, cortical shell 262 and a filler 264. Preferably, filler 264 is harvested from a femoral head, and thus naturally includes integral cancellous and cortical bone portions 266, 268, respectively. The natural geometry of cortical bone portion 268 is curved or otherwise variable at free end 270, and a flat free end 270 is not necessary to provide sufficient structural integrity. In one embodiment, filler 264 may be harvested from a femoral head, although it is difficult to obtain a natural, uniformly thick cortical bone portion 268. Preferably, the distance L₃ from a free end of cortical shell 262 to a hole 272 is chosen so that cortical bone portion 268 does not intersect any holes 272, and filler 264 may then prevent the release of blood or other fluids from within the plug or vacancy in which it is inserted. Optionally, a cortical cap may be disposed proximate free end 270; cortical bone portion 268 of filler 264 may serve as a redundant cortical sealing for plug 260 should the cap become detached.

Another alternate embodiment of a plug 280 includes a shell or sleeve 282 with holes 284 and threading 286 on outer surface 288. Plug 280 is thus configured to be threadably received in an anatomical vacancy in bone tissue. Threading on the inner surface of plug 280 also may be provided.

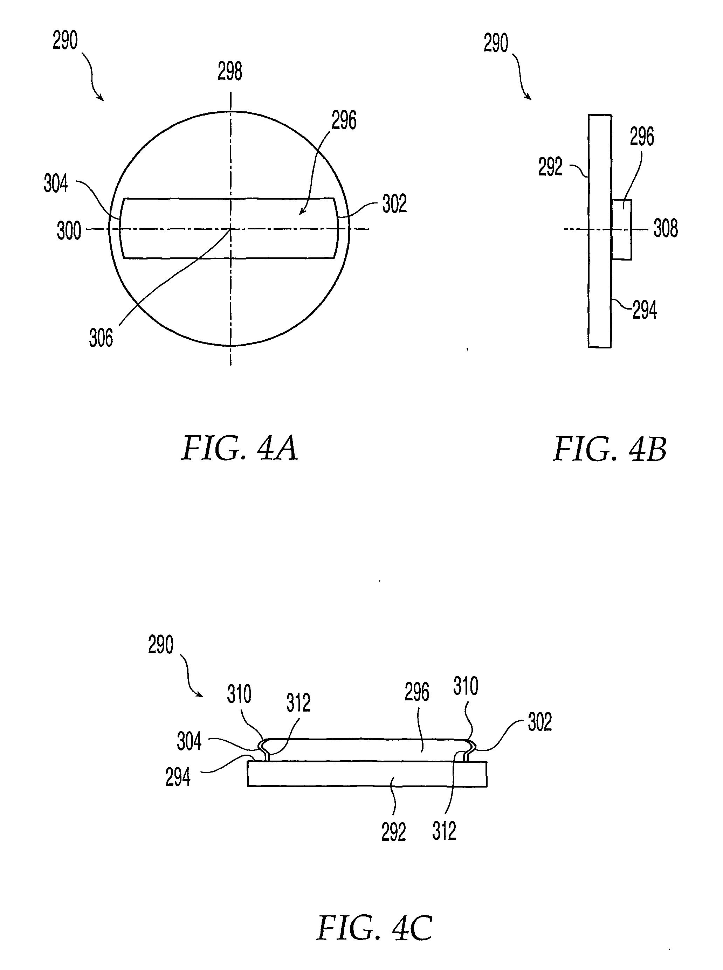

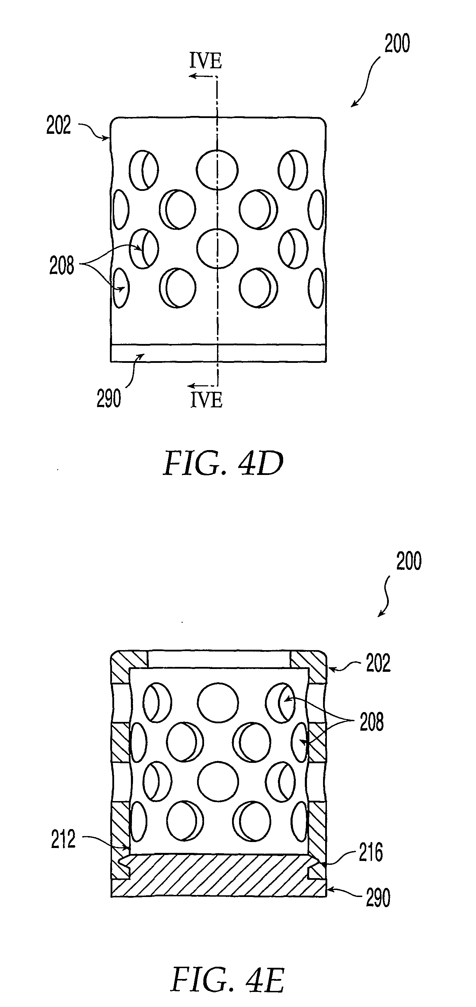

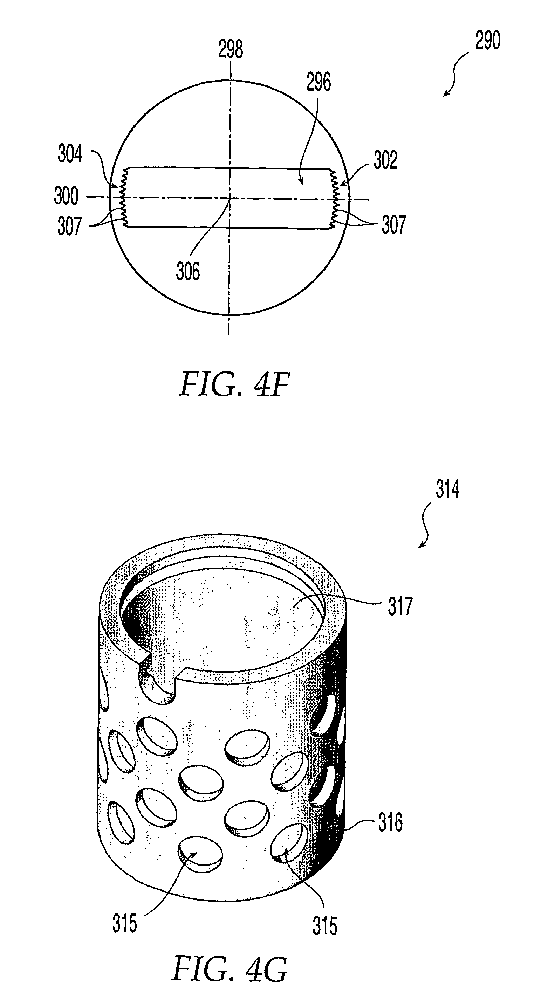

Another embodiment of a cortical cap suitable for the present invention is shown initially in FIGS. 4A to 4C. Cap 290 includes a top surface 292 and a bottom surface 294. A locking portion 296 preferably is integrally formed on bottom surface 294, and is symmetrical about central lines 298, 300. The outer edges 302, 304 of locking portion 296 are preferably disposed along the circumference of a circle centered about point 306 along central axis 308 of cap 290. As shown in FIG. 4C, locking portion 296 of cap 290 includes a lip 310 and a wall 312. As discussed with respect to cap 222, lip 310 of cap 290 is sized to fit and turn within groove 216 in sleeve 202, while wall 312 is sized to fit and turn proximate inner surface 212 of sleeve 202. A cap 290 may be coupled to a sleeve 202, preferably by snap-fitting of lip 310 of cap 290 in groove 216 of sleeve 202, as shown in FIGS. 4D and 4E. The snap-fitting of cap 290 to sleeve 202 obviates the need for a slot 214 in sleeve 202. The hole pattern of sleeve 202 may be varied, and different hole patterns are shown in FIGS. 3A and 4D. Alternate embodiments of cap 290 may include serrations, ribbing, scoring, or other undulating features on edges 302, 304 of locking portion 296, such as ribs 307 shown in FIG. 4F. Alternatively, such ribs may be oriented in a plane perpendicular to line 308, following the curvature of edges 302, 304, as shown in Fig. 4B. Ribs 307 may be used to lower tolerances between edges 302, 304 and receiving surfaces on a sleeve. Additionally, the grooving in a sleeve for receives edges 302, 304 may be provided with similar undulating features for positive interlocking.

An alternate sleeve 314 for use in a plug is shown in FIG. 4G. Sleeve 314 includes recesses 315 formed in outer surface 316. Recesses 315 do not extend through inner surface 317. Although recesses 316 are shown in the shape of circular depressions, the recesses may be other shapes such as dimples formed from a spherical geometry, diamond shapes, or rectangular shapes.

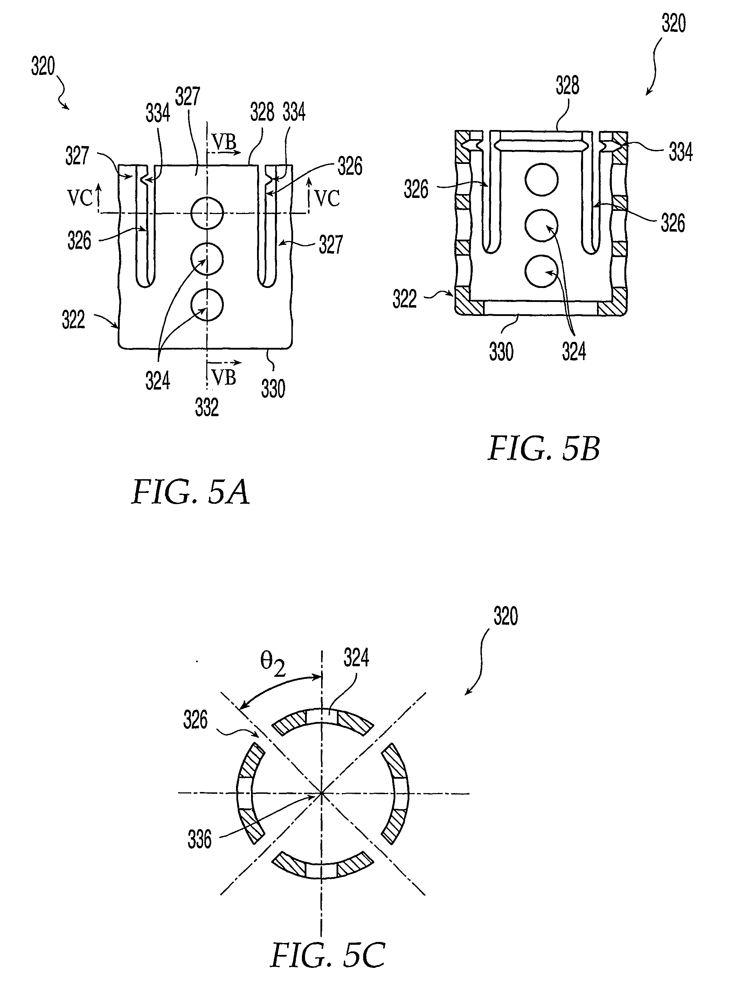

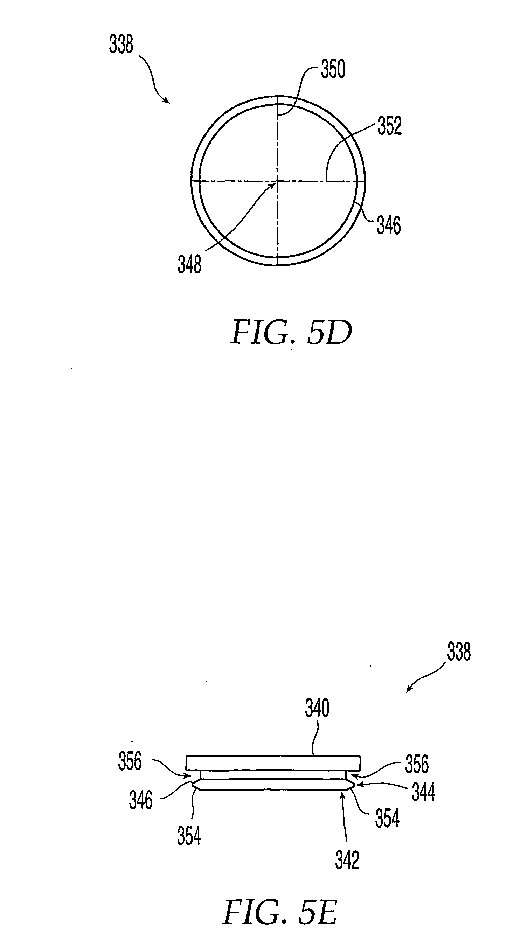

A further embodiment of a plug is shown in FIGS. 5A to 5C. Plug 320 includes a sleeve 322 with holes 324 and elongate slots 326 extending from free end 328 toward free end 330. Due to the positioning of slots 326, fingers 327 are formed. Advantageously, fingers 327 provide flexibility at free end 328, thus facilitating the snap-fitting of a cap on free end 328. Preferably, holes 324 are disposed in aligned groups, such as along line 332. A circumferential groove 334 is provided to facilitate coupling of a cap to sleeve 322, as discussed previously. Preferably, holes 324 and grooves 326 are disposed at a generally constant angular interval θ₂ with respect to each other, as measured from the center of sleeve 322 at point 336. Preferably, angle θ₂ is between about 35° and about 55° and more preferably about 45°. Another cap particularly suitable for use with plug 320 is cap 338, shown in FIGS. 5D and 5E. Cap 338 includes a top surface 340 and a bottom surface 342. A locking portion 344 preferably is integrally formed on bottom surface 342, and is circular. The outer edge 346 of locking portion 344 is disposed along the circumference of a circle centered about point 348, formed at the intersection of diameters 350, 352. Referring to FIG. 5E, locking portion 344 of cap 338 includes a lip 354 and a wall 356. As discussed with respect to other embodiments of caps, lip 354 of cap 338 is sized to fit within groove 334 in sleeve 322, while wall 356 is sized to fit proximate the inner surface of sleeve 322.

Caps referred to herein may be coupled to sleeves using a variety of other structures, including threading, ribs, teeth, tapers, and knurled surfaces. In addition, the use of adhesive bonding is also contemplated.

Although circular, elongate, and irregular protrusions have been disclosed for use in coupling caps to sleeves, other configurations of protrusions are also contemplated for use in the present invention. For example a protrusion with a triangular or rectangular geometry may be used to provide three or four points of contact, respectively, with the inner walls of a sleeve. A cross-shaped protrusion would also provide four regions of contact. In one embodiment, a rectangular protrusion is provided with a length that is a factor of three greater than the width.

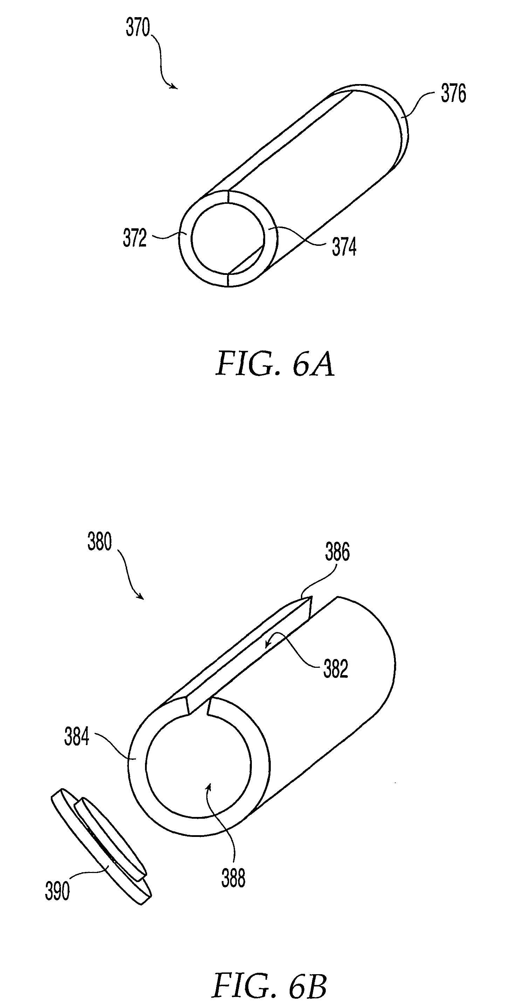

With respect to sleeve constructions, multipiece sleeves are also contemplated. For example, as shown in FIG. 6A, a sleeve 370 may be formed from two portions of bone 372, 374 that are interfitted together and closed at one end using a cap 376. Another sleeve 380 is shown in FIG. 6B, and includes a slit 382 extending from free end 384 to free end 386. Slit 382 is sized such that when a cylindrical filler is inserted into hollow region 388, sleeve 380 is permitted to flex to accommodate dimensional differences between the inner diameter of sleeve 380 and the outer diameter of the filler. Ends 384, 386 may be closed using caps, as discussed previously, for example with a cap 390. Cap 390 may optionally be provided with a slit extending from a central point to the outer diameter of the cap. Sleeve 380 also may be compressed when inserted in a vacancy, such as in a vertebral body; after insertion, sleeve 380 may re-expand to more closely fit against the walls of the vacancy. Also, sleeve 380 may be inserted into a vacancy and expanded using a wedge or pin that is subsequently inserted into slit 382.

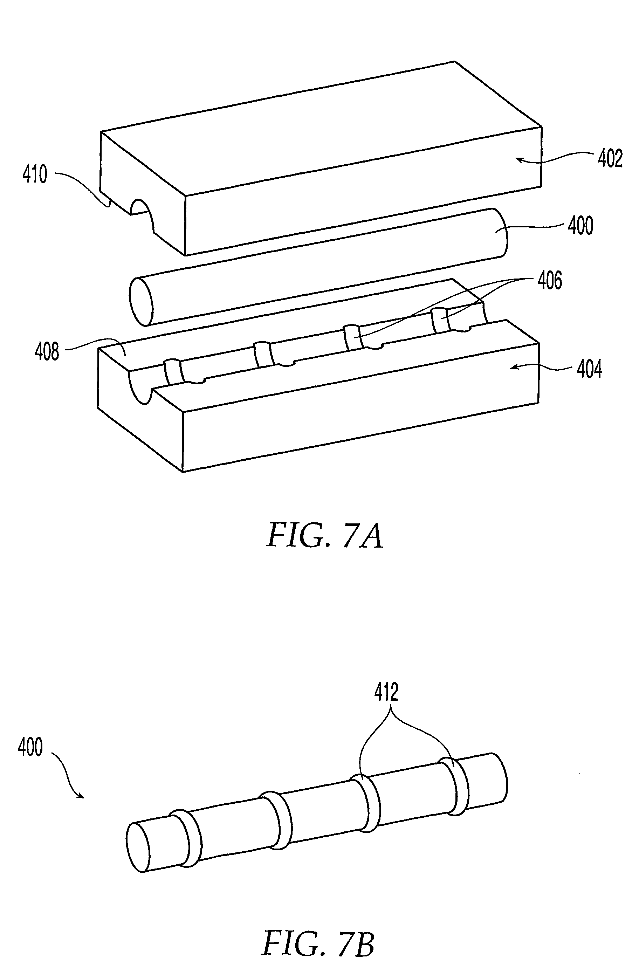

With respect to cancellous fillers for sleeves, or plugs formed of cancellous bone, the cancellous bone may be shaped to a desired geometry using dies, as shown in FIGS. 7A and 7B. Such a manufacturing process provides an alternative to turning or milling, which may be more difficult than pressing for cancellous bone. A section of cancellous bone 400 is disposed between a pair of dies 402, 404, which may include particular geometrical features such as indentations 406, protrusions, or other shapes. Once dies 402, 404 are brought together such that surfaces 408, 410 contact each other, an oversized bone section 400 is compressed and molded into the geometry of the dies. As shown in FIG. 7B, compression of a bone section 400 that is initially smooth and cylindrical in dies 402, 404 results in the formation of ribs 412 about the circumference of bone section 400 due to the compression of portions of bone section 400 in indentations 406. Thus, machining of such cancellous bone fillers may be avoided.

Advantageously, plugs formed of cancellous bone may be attached to syringes or aspirators, and blood or other fluids such as bone-growth inducing substances may be drawn into the plugs. The use of mechanically applied pressure, such as with aspiration devices, permits a greater degree of fluid absorption and/or concentration to be achieved than otherwise readily obtainable by soaking bone in such fluids without applying pressure from a device. In embodiments of the present invention that include hollow regions, a bone component of cancellous bone formed using the aforementioned technique may be inserted therein.

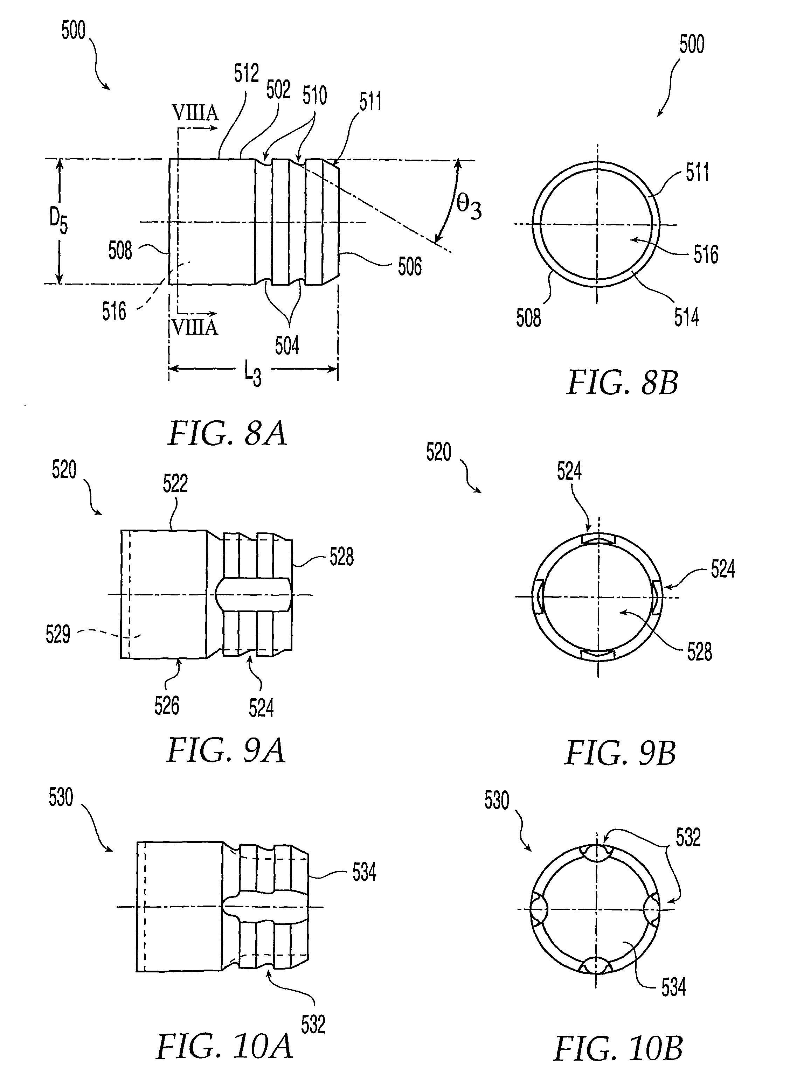

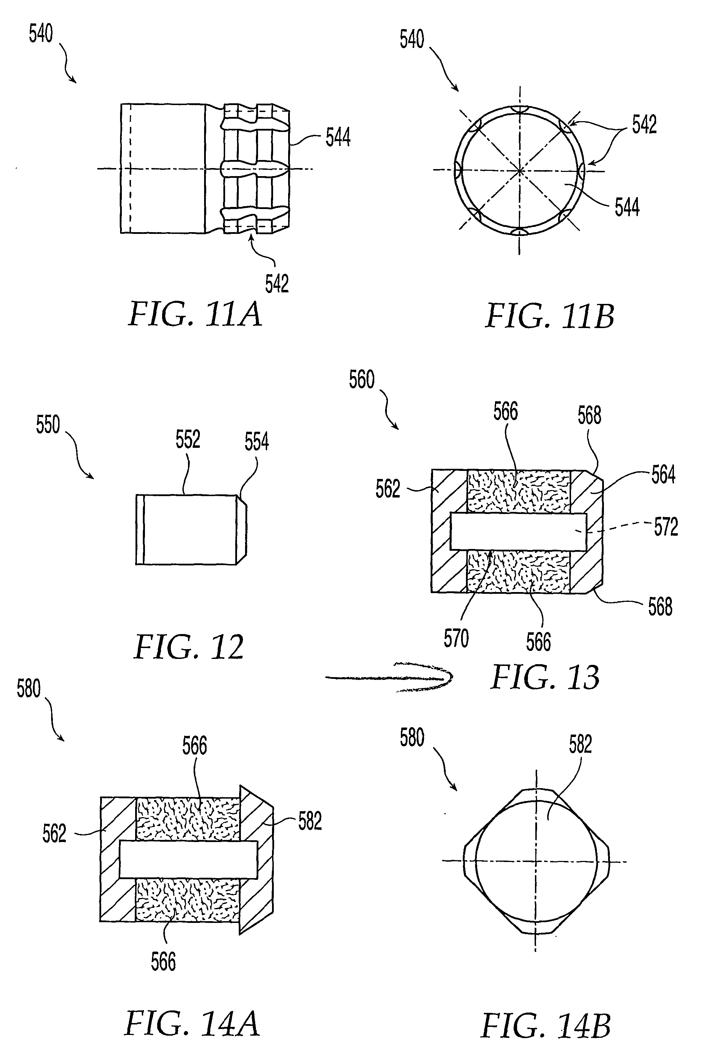

Other plugs in the form of dowels are shown in FIGS. 8 to 15. Plugs described and depicted as sleeves alternatively may be formed as solid plugs with the outer contours shown in the figures. In the preferred embodiments, the plugs are generally cylindrical. Turning to FIG. 8A, a plug 500 includes a sleeve or solid portion 510 formed of a single piece of bone. Grooved regions 504 are provided proximate free end 506, and preferably include angled edges 510 disposed at an angle θ₃ with respect to outer surface 512. Preferably, angle θ₃ is between about 20° and about 40°, and more preferably about 30°. Preferably, plug 500 has an overall length L₃ of between about 1.7 cm to about 2.3 cm, and more preferably about 2.0 cm, while the outer diameter D₅ is between about 1.4 cm and about 1.8 cm, and more preferably about 1.6 cm. In one embodiment, plug 500 is formed from bone harvested from a condyle. Thus, bone proximate free end 508 may be cortical, while the remainder of plug 500 is formed of cancellous bone. As shown in the side view of FIG. 8B, free end 506 of sleeve portion 510 has a circular wall 514 with a chamfer 511 to facilitate insertion of plug 500 into a vacancy. Similarly, a plug 520 shown in FIGS. 9A and 9B is formed of a sleeve or solid portion 522 with flutes 524 disposed about the outer surface 526 of portion 522 proximate a free end 528, as partially indicated in phantom. If plugs 500, 520 are formed as sleeves, central chambers 516, 529, respectively are formed therein. Additional forms of fluting for similarly dimensioned plugs are shown in FIGS. 10 to 11. Plugs 530, 540 include flutes 532, 542 respectively. Side views from ends 534, 544 of plugs 530, 540, respectively, are shown in FIGS. 10B and 11B.

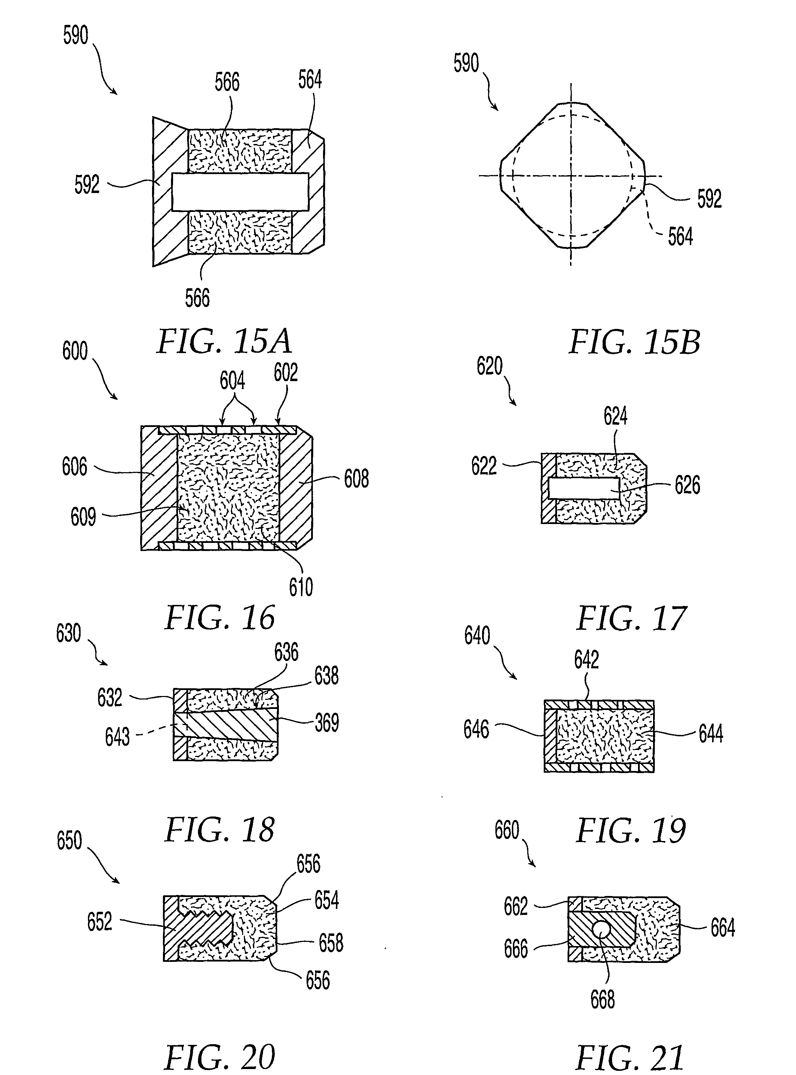

Plug 550, shown in side view in FIG. 12, includes a sleeve 552 with a chamfered end 554. As shown in FIG. 13 in cross-section, a generally cylindrical plug 560 includes cortical end caps 562, 564 disposed at the ends of a cancellous sleeve 566. End cap 564 includes chamfered edges 568. A central chamber 570 is also formed, and may be filled with a cylindrical element 572 formed of cortical bone in the shape of a pin to improve structural integrity. Preferably, element 572 is press-fit within sleeve 566 and end caps 562, 564. Similarly, as shown in FIGS. 14A and 14B, plug 580 includes an oversized end cap 582, while shown in FIGS. 15A and 15B is a plug 590 with an oversized end cap 592. The components of plugs 560, 580, 590 may be may be held together using ribbing, keys, pins, or other features as described previously with respect to other embodiments.

Additional generally cylindrical plugs are shown in cross-section in FIGS. 16-25. Turning to FIG. 16, plug 600 includes a cortical sleeve 602 with through-holes 604, cortical end caps 606, 608, and a chamber 609 with a cancellous central filler 610 disposed therein. In an alternate embodiment, because end caps 606, 608 create a chamber 609, plug 600 may be filled with other materials such as bone chips. End caps 606, 608 may be press-fit or snapped within sleeve 602. Plug 620, as shown in FIG. 17, includes a cortical cap 622 and a cancellous body 624, which together form a central, substantially right cylindrical chamber 626. Plug 630, shown in FIG. 18, includes a cortical cap 632 with a central through-hole 634, aligned with a cancellous sleeve 636 with a central through-hole 638. Holes 634, 638 are aligned such that a suitably sized pin 639 extends therethrough. In some embodiments, holes 634, 638 form a tapered hole such that a generally frustoconical chamber is formed for receiving a like-shaped filler such as a cancellous bone filler.

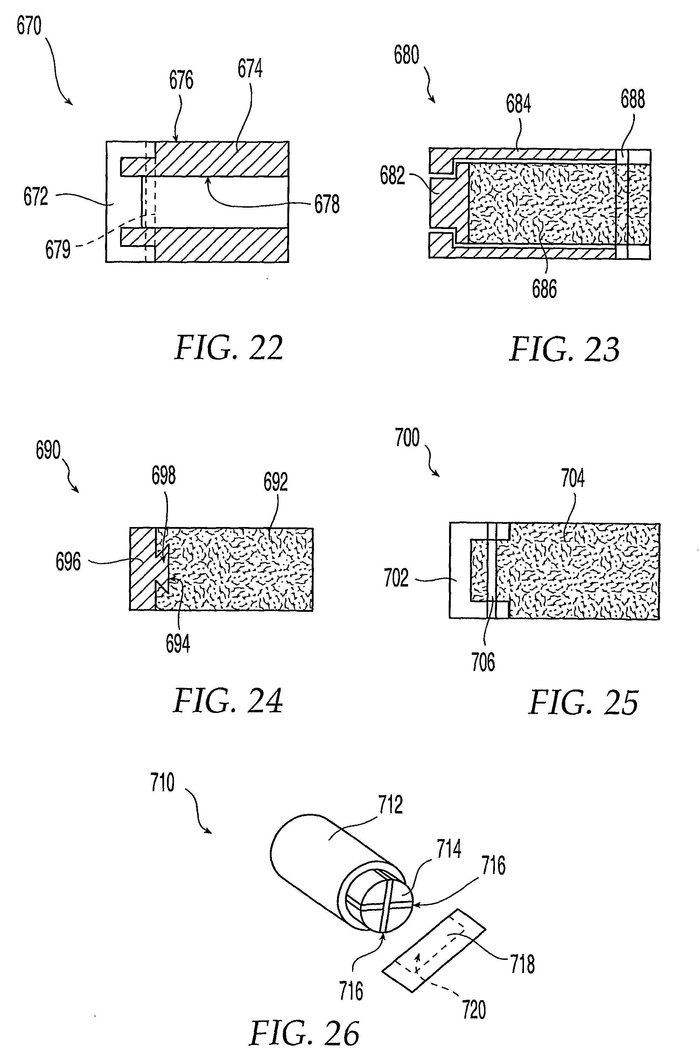

As shown in Fig. 19, a plug 640 includes a cortical sleeve 642 with a substantially right cylindrical cancellous insert 644; sleeve 642 is closed at one end with a cortical end cap 646. Plug 650 includes a cortical end cap 652 that is threadably received in a cancellous body 654. A chamfer 656 is provided at face 658, opposite end cap 652, to assist in guiding the insertion of plug 650 into a vacancy. A plug 660 includes a cortical end cap 662 and a cancellous body 664. A cortical insert 666 extends through end cap 552 and partway through body 664. Insert 666 is fixed to body 664 with a pin 668 which extends through body 664 and insert 666. Pin 668 is shown extending generally perpendicular to the plane of the page. In the embodiment shown in FIG. 22, plug 670 includes a cap 672 that press fits to sleeve 674 along both the outer and inner surfaces 676, 678, respectively. Optionally cap 672 may be pinned in place with a pin 679 extending through both cap 672 and sleeve 674. Additionally, as shown in FIG. 23, a plug 680 includes a cortical cap 682 that is locked within sleeve 684 by a cancellous insert 686. Components 682, 686 may instead be integral, formed for example by machining a potion of bone harvested from a femoral head, as described previously with respect to plug 260. A pin 688 further constrains movement of cancellous insert 686. In yet another embodiment, a sleeve 692 of plug 690 is provided with a dovetail-shaped through-slot 694 at one end so that a cap 696 may be coupled to sleeve 692 by sliding dovetail portion 698 within slot 694 from either end of the slot. Also, a plug 700 may be formed by press-fitting or loosely fitting a cortical cap 702 to a cancellous body 704 and further coupling cap 702 to body 704 with a pin 706 as shown in FIG. 25.

Turning to FIG. 26, plug 710 includes a body 712 with a protrusion 714 having slots 716. When a cap 718 with a central hole 720 is placed around protrusion 714, the protrusion may contract so that the width of slots 716 decreases, thereby providing a tighter fit of cap 718 to an oversized protrusion 714.

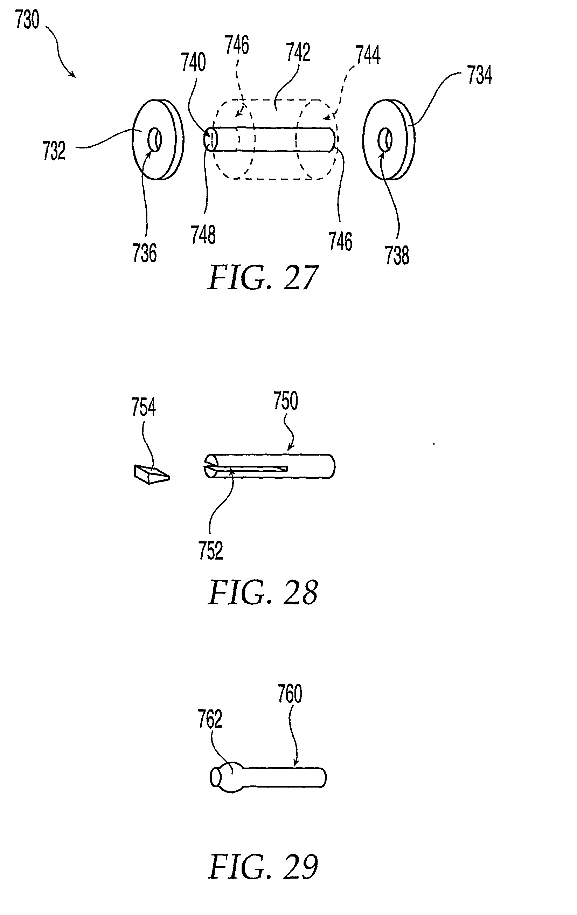

Another plug 730 is shown in exploded perspective view in FIG. 27. Washer-like end caps 732, 734 include central holes 736, 738, respectively, for receiving a central member 740. Preferably, end caps 732, 734 and central member 740 are formed of cortical bone. Member 740 also passes through a central hole in body 742, extending beyond each free end 744, 746 thereof, such that holes 736, 738 receive portions of member 740. Preferably, member 740 includes interlocking features at free ends 746, 748 to facilitate coupling. Such features may include a taper, ribs, threads, saw teeth, flanges, or knurls. In one embodiment, shown in exploded perspective view in FIG. 28, a member 750 suitable for use with plug 730 includes a slotted portion 752 and an insertable wedge-shaped portion 754. Rod 750 is initially disposed in an end cap hole such as hole 736, without wedge-shaped portion 754. Rod 750 is locked in place to end cap 732 by inserting wedge-shaped portion 754 in slot 752 and thus increasing the effective diameter of rod 750 so that a press-fit within hole 736 is achieved. Similarly, a press-fit may be achieved using a member 760 with a bulging end 762, as shown in the perspective view of FIG. 29. Insertion of bulging end 762 in a hole 736, for example, provides a fit such that member 760 may be coupled to end cap 732. Such a member 760 may be in the form of a button snap mechanical fastener.

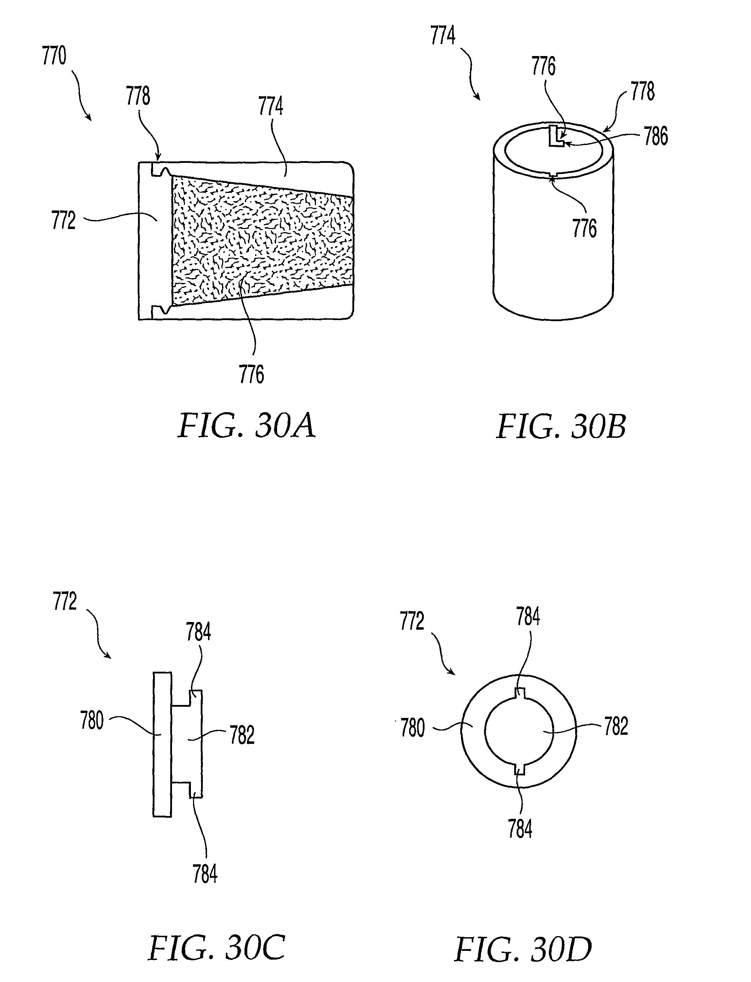

Yet another plug 770 is shown in FIGS. 30A to 30D. As shown in the cross-sectional side view of FIG. 30A, plug 770 includes end cap 772, tapered sleeve 774, and tapered insert 776 sized to fit in sleeve 774. A pair of L-shaped recesses 776 are disposed proximate end 778, as shown in the perspective view of FIG. 30B. As shown in side view in FIG. 30C and bottom view in FIG. 30D, end cap 772 includes head 780 and shaft 782. A pair of opposing protrusions 784 are disposed on shaft 782. Protrusions 784 are located and sized on shaft 782 such that end cap 772 may be inserted onto end 778 of sleeve 774 with protrusions 784 fitting in L-shaped recesses 776. To fix end cap 772 to sleeve 774, end cap 772 is turned so that protrusions 784 abut edges 786 of recesses 776.

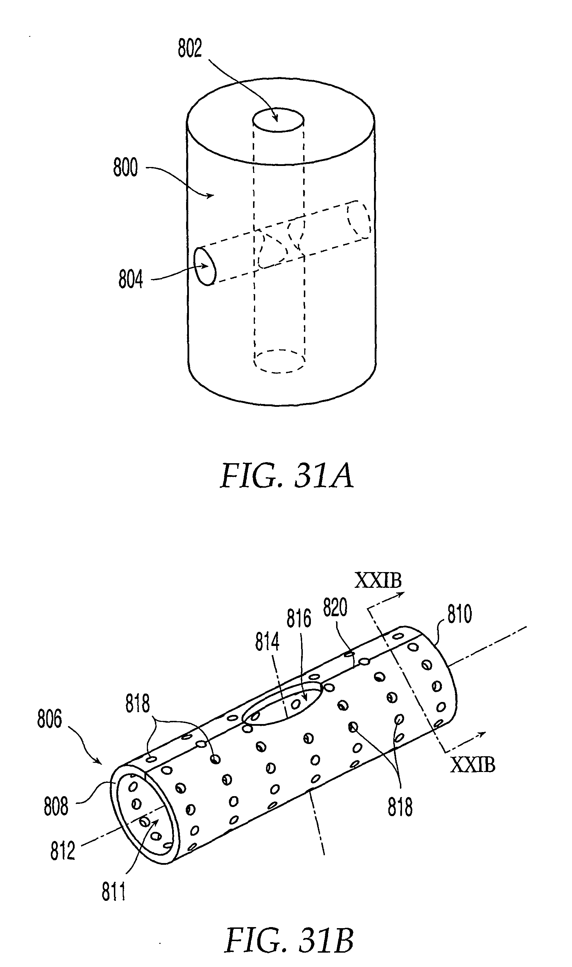

Referring now to FIG. 31A, a section 800 of a long bone such as a humerus is shown. Although the particular geometry of the inside and outside of the bone is shown as being generally cylindrical for exemplary purposes, a plug formed from section 800 in part may follow the natural outer and inner geometry of section 800. Bone 800 includes a canal 802. A generally cylindrical plug 804, taken transverse to the long axis of a bone, may be removed from bone section 800 and may be used to form a plug 806. Plug 806 includes free ends 808, 810. When initially removed from bone section 800, free ends 808, 810 are both closed. A cavity 811 may be bored into a free end 808, 810. Preferably, cavity 811 is disposed about axis 812, which is generally perpendicular to axis 814 of through-hole 816 formed by canal 802. Thus, cavity 811 is bounded by an open free end 808 and closed free end 810, forming a sleeve. Plug 806 may be filled with materials such as bone chips, bone particulate, bone fibers, bone growth materials, hydroxyapatite, metal, resorbable material, polymer, ceramic, and bone cement. Preferably, perforations 818 are formed in plug 806. A cap (not shown) also may be fixed to free end 808. Preferably, alignment indicia 820 is provided such as a line extending from free end 808 to free end 810 and across through-hole 816. Free end 810, which is closed, also may include indicia. Such indicia may facilitate positioning of plug 806 by a surgeon, particularly with respect to the orientation of through-hole 816 in the anatomical vacancy to be filled. In addition, it should be noted that a section taken through line XXIB-XXIB may create an end cap such as cap 718 shown in FIG. 26.

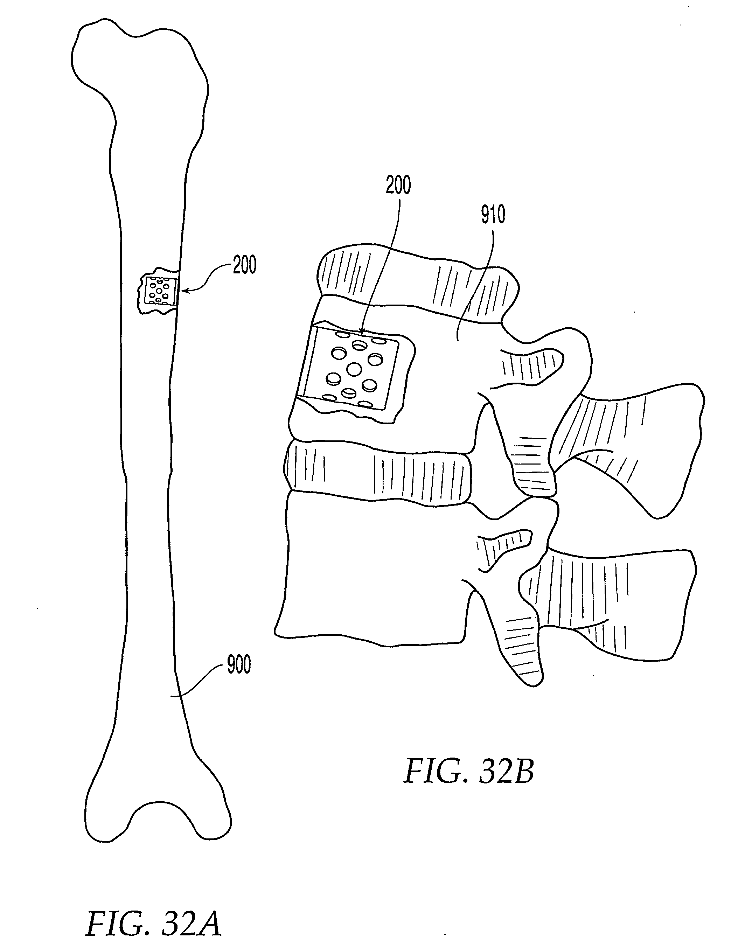

The plugs disclosed in the present invention may be sized to meet a particular need and applied in areas of bony tissue. Exemplary use of the plugs described herein is shown in FIGS. 32A and 32B. A plug 200, for example, may be used in a bone such as a long bone 900. In addition, a plug 200 may be used in a vertebral body 910. Plugs 200 are used to fill voids that may exist or be created in long bone 900 and vertebral body 910 due to trauma, disease, malformation, or other conditions. Also, the plugs may be used to fill holes in healthy bone tissue that are surgically created when healthy tissue is removed for transplantation to other bony regions of the body.

The embodiments of plugs disclosed herein may include components that are initially provided with a first moisture content, but then allowed to assume a new configuration with a second moisture content. For example, in the embodiment shown in FIG. 4E, a cap 290 is used with a sleeve 202. Cap 290 may have a first outer diameter for lip 310. Freeze-drying of cap 290 results in shrinkage such that lip 310 assumes a configuration with a second outer diameter that is smaller than the first outer diameter. When cap 290 is rehydrated or treated with a swelling agent, lip 310 of cap 290 may reassume a configuration with the first outer diameter. Plug 200 initially may be provided with a freeze-dried cap 290 disposed inside another bone section such as sleeve 202 so that a loose interference fit is achieved, and subsequent rehydration of cap 290 in place permits a tighter interference fit. Notably, the shrinkage and expansion effects may be used with bone components that have both outer and inner diameters, such as sleeve 202.

For example, sleeve 202 initially may be supplied with a first outer diameter and a first inner diameter, and subsequently freeze-dried so that sleeve 202 assumes a configuration with a second outer diameter that is smaller than the first outer diameter, while having a second inner diameter that is smaller than the first inner diameter. When sleeve 202 is rehydrated or treated with a swelling agent, sleeve 202 may reassume a configuration with the first outer diameter and a first inner diameter. Thus, sleeve 202 may first be provided in dehydrated state to loosely fit in a void, and rehydrated after insertion to provide a tighter interference fit between the sleeve and its anatomical surroundings. Use of these properties also can permit greater variation in dimensional tolerance between bone sections during manufacture, while tight final assembly can still be achieved. In addition, protrusions on bone sections become smaller when dehydrated, but expand when rehydrated; in contrast, recesses in bone sections become smaller when hydrated, but larger when dehydrated. Furthermore, temperature changes may be used to achieve better interference fits.

Alignment indicia 285 such as a line along the side of sleeve 280, as shown in FIG. 3N, may be provided on the outer surface of the sleeve. Preferably, indicia 285 is an imprint, i.e. with ink, although indicia 285 may instead be provided in the form of surface scoring or a protrusion on the surface. In addition to sleeves, caps may also be provided with indicia to assist in properly orienting the caps, along with any component coupled thereto, after insertion into an anatomical void. Also, the indicia may be used to properly align a plug with a particular body or sleeve hole configuration to facilitate and guide the release or exposure of substances contained therein. The indicia suitable for the present invention includes, but is not limited to, markers such as lines, arrows, lettering, and symbols.

Numerous types of joints are useful in the present development, including joints that permit articulation such as a ball and socket type of joint, and particularly joints that permit firm interlocking between two components to prevent relative movement between the components. Preferably, mortise and tenon joints can be used to interfit components of the plugs. Other coupling arrangements such as edge joints including tongue and groove joints, rabbeted joints, toothed joints, and dovetail joints are also suitable for the present invention.

The use of insertable securing elements such as keys, pegs, pins, wedges, or other suitable components in joints to assist in securing bone components to each other is also an effective approach to providing a stable joint. Keys, for example, may be inserted in notched or grooved areas in plug components, serving as the securing element between two or more plug components. Parameters that may be varied when using insertable securing elements, such as keys, include the angle of application, the spacing of the elements, and the thicknesses of the elements.

While various descriptions of the present invention are described above, it should be understood that the various features can be used singly or in any combination thereof. The various types of joints and connections can be used on plugs of different size or configuration, such that the invention is not to be limited to only the specifically preferred embodiments depicted in the drawings.

Further, it should be understood that variations and modifications within the scope of the invention may occur to those skilled in the art to which the invention pertains. For example, multiple, differently shaped and sized plugs can be constructed for interfitting or interconnection to form a multiple part plug that serves the desired purpose.

The scope of the present invention is accordingly defined as set forth in the appended claims.

## Claims

1. A plug (200) for filling a vacant region in anatomical bone, comprising:
a body with a top end (204), a bottom end (206), and an outer surface disposed between the top and bottom ends (204,206); and
a first cap (222),
wherein the body and first cap (222) are substantially formed from bone selected from the group consisting of cortical and cancellous bone, **characterized in that** said first cap (222) comprises a locking portion (228) mechanically lockable to an end of the body and configured and dimensioned to be rotatably received in the body,
wherein the locking portion (228) extends substantially across a face of the first cap (222).

2. The plug (200) of claim 1, wherein the body comprises a sleeve (202) and an inner surface.

3. The plug (200) of claim 2, further comprising an insert configured and dimensioned to be received in the sleeve (202).

4. The plug (200) of claim 3, wherein the insert is formed of cancellous bone.

5. The plug (200) of claim 4, wherein the cancellous bone has a fluid concentrated therein.

6. The plug (200) of claim 5, wherein the insert is subjected to mechanical pressure to concentrate the fluid.

7. The plug (200) of claim 6, wherein the mechanical pressure is applied by aspiration.

8. The plug (200) of claim 5, wherein the fluid is concentrated by soaking.

9. The plug (200) of claim 4, wherein the insert is secured to at least one of the sleeve (202) and first cap (222) with at least one fastener.

10. The plug (200) of claim 9, wherein the at least one fastener is selected from a screw, key, pin, peg, rivet, cotter, nail, spike, bolt, stud, staple, boss, clamp, clip, dowel, stake, hook, anchor, tie, band, crimp and wedge.

11. The plug (200) of claim 10, wherein at least one of the sleeve (202), first cap (222), insert, and fastener is formed from partially demineralised or demineralised bone.

12. The plug (200) of claim 9, wherein at least two of the sleeve (202), first cap (222), insert, and fastener are bonded together with a bonding agent.

13. The plug (200) of claim 9, wherein at least one of the sleeve (202), first cap (222), insert, and fastener is at least partially dehydrated to loosely fit within a surrounding mating surface.

14. The plug (200) of claim 4, wherein at least one of the sleeve (202), first cap (222), and insert further comprises alignment indicia.

15. The plug (200) of claim 2, wherein the sleeve (202) is packed with at least one of bone chips, bone particulate, bone fibers, bone growth materials, hydroxyapatite, metal, resorbable material, polymer, ceramic, and bone cement.

16. The plug (200) of claim 15, wherein the sleeve (202) further comprises at least one through-hole (208) extending from the inner surface to the outer surface.

17. The plug (200) of claim 16, wherein the sleeve (202) is cylindrical.

18. The plug (200) of claim 15, wherein the sleeve (202) further comprises at least one depression extending from the outer surface toward the inner surface.

19. The plug (200) of claim 2, wherein the sleeve (202) further comprises a plurality of fingers (327) formed integrally therewith.

20. The plug (200) of claim 2, wherein the sleeve (202) and first cap (222) are substantially formed of cortical bone.

21. The plug (200) of claim 2, wherein the body is formed of cancellous bone and the first cap is formed of cortical bone.

22. The plug (200) of claim 2, wherein one of the top and bottom ends (204,206) of the body is open.

23. The plug (200) of claim 1, further comprising a second cap disposed on a different end from the first cap.

24. The plug (200) of claim 1, further comprising:
a sleeve (202) comprising the top end (204), the bottom end (206), an inner surface, the outer surface disposed between the top and bottom ends (204,206), a groove (216) disposed on the inner surface proximate one of the ends (204,206), and a plurality of through-holes (208) extending from the inner surface to the outer surface;
the first cap (222) comprising opposing faces (224,226), the locking portion (228) configured and dimensioned to be received in the groove (216); and
wherein one of the faces (224,226) of the first cap (222) abuts one of the ends of the sleeve (202).

25. The plug (200) of claim 24, wherein the sleeve (202) and first cap (222) are substantially formed of cortical bone.

26. The plug (200) of claim 24, wherein the sleeve (202) is formed of cancellous bone and the first cap (222) is formed of cortical bone.

27. The plug (200) of claim 24, wherein one of the top and bottom ends (204,206) of the body if open.

28. The plug (200) of claim 24, wherein the sleeve (202) further comprises threading (286) disposed on the outer surface thereof.

29. The plug (200) of claim 24, wherein the sleeve (202) further comprises a slot (214) intersecting the groove (216).

30. The plug (200) of claim 29, wherein the slot (214) has a width sized to allow passage of a portion of the locking portion (228) of the cap.

31. The plug (200) of claim 24, wherein the outer surface of the sleeve (202) is substantially cylindrical.

32. The plug (200) of claim 24, wherein the outer surface of the sleeve (202) is substantially arcuate.

33. The plug (200) of claim 29, wherein the sleeve (202) further comprises a central axis and a circumferential lip (236) extending toward the central axis.

34. The plug (200) of claim 33, wherein the circumferential lip (236) is disposed proximate an end of the sleeve (202).

35. The plug (500) of claim 24, wherein the sleeve further comprises a chamfer (511) proximate an end of the sleeve (510).

36. The plug (200) of claim 24, wherein the locking portion (228) is integrally formed with the first cap (222).

37. The plug (200) of claim 24, wherein the locking portion (228) comprises a substantially arcuate outer edge.

38. The plug (200) of claim 24, wherein the locking portion (228) comprises a substantially circular outer edge.

39. The plug (200) of claim 24, wherein the locking portion (296) comprises an outer edge provided with undulations.

40. The plug (200) of claim 39, wherein the undulations form a cerrated edge.

41. The plug (200) of claim 24, wherein the locking portion (228) is generally symmetrical about a central line and tapers from a wide portion (232) to a narrow portion (230).

42. The plug (200) of claim 41, wherein the locking portion (228) tapers from the wide portion (232) to the narrow portion (230) along a radius of curvature of between 0.5 cm and 1.1 cm.

43. The plug (200) of claim 41, wherein outer edges of the narrow and wide portions (230,232) are disposed substantially along the circumference of a circle.

44. The plug (200) of claim 24, wherein the locking portion (228) is disposed on one of the faces (224,226) of the first cap (222).

45. The plug (200) of claim 24, wherein the first cap (222) further comprises a first outer diameter and the sleeve (202) further comprises a second outer diameter, the first and second outer diameters being the same.

46. The plug (320) of claim 24, wherein the sleeve further comprises a plurality of fingers (327) formed integrally therewith.

47. The plug (200) of claim 24, further comprising a second cap disposed on a different end from the first cap (222).

48. The plug (200) of claim 24, wherein the sleeve (202) further comprises a stop for limiting rotation of .the locking portion (228) of the first cap (222) when disposed in the groove (216).

49. The plug (200) of claim 48, wherein the stop interrupts the groove (216).

50. The plug (200) of claim 24, wherein the groove (216) extends circumferentially around the inner surface of the sleeve (202).

51. The plug (200) of claim 24, further comprising an insert formed of bone and configured and dimensioned to be received in the sleeve (202).

52. The plug (600) of claim 24, wherein the through-holes (604) are arranged in a plurality of rows.

53. The plug (600) of claim 52, wherein the rows are disposed in generally parallel planes.

54. The plug (600) of claim 52, wherein the through-holes (604) are disposed at a generally constant angular interval with respect to each other as measured from a central axis of the sleeve.

## Patentansprüche

1. Pfropfen (200) zum Füllen eines leeren Bereichs in anatomischen Knochen, umfassend:
einen Körper mit einem oberen Ende (204), einem unteren Ende (206) und einer äußeren Oberfläche, welche zwischen dem oberen und dem unteren Ende (204, 206) angeordnet ist; und
eine erste Kappe (222),
wobei der Körper und die erste Kappe (222) im Wesentlichen aus Knochen gebildet sind, welcher ausgewählt ist aus der Gruppe bestehend aus kortikalem und sponginösem Knochen,
**dadurch gekennzeichnet, dass**
die erste Kappe (222) einen Verschlussabschnitt (228) umfasst, welcher mit einem Ende des Körpers mechanisch verschließbar ist und derart konfiguriert und bemessen ist, um in dem Körper drehbar aufgenommen zu werden;
wobei der Verschlussabschnitt (228) sich im Wesentlichen über eine Seite der ersten Kappe (222) erstreckt.

2. Pfropfen (200) nach Anspruch 1, wobei der Körper eine Hülse (202) und eine innere Oberfläche umfasst.

3. Pfropfen (200) nach Anspruch 2, ferner umfassend einen Einsatz, welcher derart konfiguriert und bemessen ist, um in der Hülse (202) aufgenommen zu werden.

4. Pfropfen (200) nach Anspruch 3, wobei der Einsatz aus sponginösem Knochen gebildet ist.

5. Pfropfen (200) nach Anspruch 4, wobei der sponginöse Knochen ein Fluid darin konzentriert aufweist.

6. Pfropfen (200) nach Anspruch 5, wobei der Einsatz mechanischem Druck ausgesetzt worden ist, um das Fluid zu konzentrieren.

7. Pfropfen (200) nach Anspruch 6, wobei der mechanische Druck durch Aspiration erzeugt worden ist.

8. Pfropfen (200) nach Anspruch 5, wobei das Fluid durch Einweichen konzentriert worden ist.

9. Pfropfen (200) nach Anspruch 4, wobei der Einsatz an wenigstens einer von Hülse (202) und erster Kappe (222) mit wenigstens einem Befestigungsmittel gesichert ist.

10. Pfropfen (200) nach Anspruch 9, wobei das wenigstens eine Befestigungsmittel ausgewählt ist aus Schraube, Schlüssel, Stift, Zapfen, Niete, Splint, Nagel, Dorn, Bolzen, Knopf, Heftklammer, Nabe, Klemme, Klammer, Dübel, Pflock, Haken, Anker, Schnur, Band, Falz und Keil.

11. Pfropfen (200) nach Anspruch 10, wobei von der Hülse (202), der ersten Kappe (222), dem Einsatz und dem Befestigungsmittel wenigstens eins aus teilweise demineralisiertem oder demineralisiertem Knochen gebildet ist.

12. Pfropfen (200) nach Anspruch 9, wobei von der Hülse (202), der ersten Kappe (222), dem Einsatz und dem Befestigungsmittel wenigstens zwei mittels eines Haftmittels aneinander gebunden sind.

13. Pfropfen (200) nach Anspruch 9, wobei von der Hülse (202), der ersten Kappe (222), dem Einsatz und dem Befestigungsmittel wenigstens eins wenigstens partiell dehydratisiert ist, um lose in eine umgebende Berührungsfläche hinein zu passen.

14. Pfropfen (200) nach Anspruch 4, wobei von der Hülse (202), der ersten Kappe (222) und dem Einsatz wenigstens eins ferner Ausrichtungsmarkierungen umfasst.

15. Pfropfen (200) nach Anspruch 2, wobei die Hülse (202) mit wenigstens einem von Knochensplittern, Knochenpartikeln, Knochenfasern, Knochenwachstumsmaterialien, Hydroxyapatit, Metall, resorbierbarem Material, Polymer, Keramik und Knochenzement bepackt ist.

16. Pfropfen (200) nach Anspruch 15, wobei die Hülse (202) ferner wenigstens ein Durchgangsloch (208) umfasst, welches sich von der inneren Oberfläche zu der äußeren Oberfläche erstreckt.

17. Pfropfen (200) nach Anspruch 16, wobei die Hülse (202) zylindrisch ist.

18. Pfropfen (200) nach Anspruch 15, wobei die Hülse (202) ferner wenigstens eine Vertiefung umfasst, welche sich von der äußeren Oberfläche zur inneren Oberfläche hin erstreckt.

19. Pfropfen (200) nach Anspruch 2, wobei die Hülse (202) ferner mehrere integral mit ihr gebildete Finger (327) umfasst.

20. Pfropfen (200) nach Anspruch 2, wobei die Hülse (202) und die erste Kappe (222) im Wesentlichen aus kortikalem Knochen gebildet sind.

21. Pfropfen (200) nach Anspruch 2, wobei der Körper aus sponginösem Knochen gebildet ist und die erste Kappe aus kortikalem Knochen gebildet ist.

22. Pfropfen (200) nach Anspruch 2, wobei das obere oder das untere Ende (204, 206) des Körpers offen ist.

23. Pfropfen (200) nach Anspruch 1, ferner umfassend eine zweite Kappe, welche an einem anderen Ende angeordnet ist als die erste Klappe.

24. Pfropfen (200) nach Anspruch 1, ferner umfassend:
eine Hülse (202), umfassend das obere Ende (204), das untere Ende (206), eine innere Oberfläche, die äußere Oberfläche, welche zwischen dem oberen und dem unteren Ende (204, 206) angeordnet ist, eine Furche (216), welche an der inneren Oberfläche in der Nähe eines der Enden (204, 206) angeordnet ist, und mehrere Durchgangslöcher (208), welche sich von der inneren Oberfläche zur äußeren Oberfläche erstrecken;
wobei die erste Kappe (222) einander entgegengesetzte Flächen (224, 226) umfasst, der Verschlussabschnitt (228) derart konfiguriert und bemessen ist, um in der Furche (216) aufgenommen zu werden; und
wobei eine der Flächen (224, 226) der ersten Kappe (222) an eines der Enden der Hülse (202) anstößt.

25. Pfropfen (200) nach Anspruch 24, wobei die Hülse (202) und die erste Kappe (222) im Wesentlichen aus kortikalem Knochen gebildet sind.

26. Pfropfen (200) nach Anspruch 24, wobei die Hülse (202) aus sponginösem Knochen und die erste Kappe (222) aus kortikalem Knochen gebildet ist.

27. Pfropfen (200) nach Anspruch 24, wobei das obere Ende oder das untere Ende (204, 206) des Körpers offen ist.

28. Pfropfen (200) nach Anspruch 24, wobei die Hülse (202) ferner auf ihrer äußeren Oberfläche ein Gewinde (286) umfasst.

29. Pfropfen (200) nach Anspruch 24, wobei die Hülse (202) ferner einen Spalt (214) umfasst, welcher die Furche (216) schneidet.

30. Pfropfen (200) nach Anspruch 29, wobei der Spalt (214) eine Breite mit einer derarten Größe aufweist, dass der Durchtritt eines Abschnitts des Verschlussabschnitts (228) der Kappe ermöglicht ist.

31. Pfropfen (200) nach Anspruch 24, wobei die äußere Oberfläche der Hülse (202) im Wesentlichen zylindrisch ist.

32. Pfropfen (200) nach Anspruch 24, wobei die äußere Oberfläche der Hülse (202) im Wesentlichen gebogen ist.

33. Pfropfen (200) nach Anspruch 29, wobei die Hülse (202) ferner eine zentrale Achse und eine Umfangslippe (236) umfasst, welche sich zur zentralen Achse hin erstreckt.

34. Pfropfen (200) nach Anspruch 33, wobei die Umfangslippe (236) nahe einem Ende der Hülse (202) angeordnet ist.

35. Pfropfen (500) nach Anspruch 24, wobei die Hülse (202) ferner eine Abschrägung (511) nahe einem Ende der Hülse (510) umfasst.

36. Pfropfen (200) nach Anspruch 24, wobei der Verschlussabschnitt (228) integral mit der ersten Kappe (222) gebildet ist.

37. Pfropfen (200) nach Anspruch 24, wobei der Verschlussabschnitt (228) eine im Wesentlichen gebogene äußere Kante umfasst.

38. Pfropfen (200) nach Anspruch 24, wobei der Verschlussabschnitt (228) eine im Wesentlichen kreisförmige äußere Kante umfasst.

39. Pfropfen (200) nach Anspruch 24, wobei der Verschlussabschnitt (296) eine mit Wellen versehene äußere Kante umfasst.

40. Pfropfen (200) nach Anspruch 39, wobei die Wellen eine gezahnte Kante bilden.

41. Pfropfen (200) nach Anspruch 24, wobei der Verschlussabschnitt (228) im Wesentlichen symmetrisch um eine zentrale Linie ist und sich von einem weiten Abschnitt (232) zu einem engen Abschnitt (230) verjüngt.

42. Pfropfen (200) nach Anspruch 41, wobei der Verschlussabschnitt (228) sich von dem weiten Abschnitt (232) zu dem engen Abschnitt (230) entlang einem Krümmungsradius von zwischen 0,5 cm und 1,1 cm verjüngt.

43. Pfropfen (200) nach Anspruch 41, wobei äußere Kanten von dem weiten und dem engen Abschnitt (230, 232) im Wesentlichen entlang dem umfang eines Kreises angeordnet sind.

44. Pfropfen (200) nach Anspruch 24, wobei der Verschlussabschnitt (228) an einer der Flächen (224, 226) der ersten Kappe (222) angeordnet ist.

45. Pfropfen (200) nach Anspruch 24, wobei die erste Kappe (222) ferner einen ersten Außendurchmesser umfasst und die Hülse (202) ferner einen zweiten Außendurchmesser umfasst, wobei der erste und der zweite Außendurchmesser gleich sind.

46. Pfropfen (320) nach Anspruch 24, wobei die Hülse ferner mehrere mit ihr integral gebildete Finger (327) umfasst.

47. Pfropfen (200) nach Anspruch 24, ferner umfassend eine zweite Kappe, welche an einem anderen Ende angeordnet ist als die erste Kappe (222).

48. Pfropfen (200) nach Anspruch 24, wobei die Hülse (202) ferner eine Sperre zum Begrenzen der Drehung des Verschlussabschnitts (228) der ersten Kappe (222) umfasst, wenn er in der Furche (216) angeordnet ist.

49. Pfropfen (200) nach Anspruch 48, wobei die Sperre die Furche (216) unterbricht.

50. Pfropfen (200) nach Anspruch 24, wobei die Furche (216) sich ringsum die innere Oberfläche der Hülse (202) erstreckt.

51. Pfropfen (200) nach Anspruch 24, ferner umfassend einen Einsatz, welcher aus Knochen gebildet ist und derart konfiguriert und bemessen ist, um in der Hülse (202) aufgenommen zu werden.

52. Pfropfen (600) nach Anspruch 24, wobei die Durchgangslöcher (604) in mehreren Reihen angeordnet sind.

53. Pfropfen (600) nach Anspruch 52, wobei die Reihen in im Allgemeinen parallelen Ebenen angeordnet sind.

54. Pfropfen (600) nach Anspruch 52, wobei die Durchgangslöcher (604) in einem im Allgemeinen konstanten Winkelabstand hinsichtlich jedes anderen angeordnet sind, gemessen von einer zentralen Achse der Hülse.

## Revendications

1. Bouchon (200) destiné à remplir une région vacante dans un os anatomique, comprenant :
un corps avec une extrémité supérieure (204), une extrémité inférieure (206) et une surface extérieure disposée entre les extrémités supérieure et inférieure (204, 206) ; et
un premier capuchon (222)
dans lequel le corps et le premier capuchon (222) sont sensiblement formés à partir de l'os sélectionné parmi le groupe composé d'os cortical et d'os spongieux, **caractérisé en ce que** ledit premier capuchon (222) comprend une partie de verrouillage (228) pouvant être verrouillée mécaniquement à une extrémité du corps et configurée et dimensionnée pour être reçue de manière rotative dans le corps,
dans lequel la partie de verrouillage (228) s'étend sensiblement à travers une face du premier capuchon (222).

2. Bouchon (200) selon la revendication 1, dans lequel le corps comprend un manchon (202) et une surface intérieure.

3. Bouchon (200) selon la revendication 2, comprenant en outre un insert configuré et dimensionné pour être reçu dans le manchon (202).

4. Bouchon (200) selon la revendication 3, dans lequel l'insert est formé d'os spongieux.

5. Bouchon (200) selon la revendication 4, dans lequel l'os spongieux présente un fluide concentré à l'intérieur.

6. Bouchon (200) selon la revendication 5, dans lequel l'insert est soumis à une pression mécanique pour concentrer le fluide.

7. Bouchon (200) selon la revendication 6, dans lequel la pression mécanique est appliquée par aspiration.

8. Bouchon (200) selon la revendication 5, dans lequel le fluide est concentré par imbibition.

9. Bouchon (200) selon la revendication 4, dans lequel l'insert est fixé à au moins un du manchon (202) et du premier capuchon (222) avec au moins un dispositif de fixation.

10. Bouchon (200) selon la revendication 9, dans lequel le ou chaque dispositif de fixation est sélectionné parmi une vis, une clé, une goupille, un taquet, un rivet, une clavette, un clou, une pointe, un boulon, un goujon, une agrafe, un bossage, un collier de serrage, une pince, une goupille de positionnement, un tasseau, un crochet, une ancre, une attache, une courroie, un sertissage et un coin.

11. Bouchon (200) selon la revendication 10, dans lequel au moins un du manchon (202), du premier capuchon (222), de l'insert et du dispositif de fixation est formé à partir d'os partiellement déminéralisé ou déminéralisé.

12. Bouchon (200) selon la revendication 9, dans lequel au moins deux du manchon (202), du premier capuchon (222), de l'insert et du dispositif de fixation sont collés ensemble avec un agent de liaison.

13. Bouchon (200) selon la revendication 9, dans lequel au moins un du manchon (202), du premier capuchon (222), de l'insert et du dispositif de fixation est au moins partiellement déshydraté pour s'insérer de manière lâche à l'intérieur de la surface d'accouplement environnante.

14. Bouchon (200) selon la revendication 4, dans lequel au moins un du manchon (202), du premier capuchon (222) et de l'insert comprend en outre des indices d'alignement.

15. Bouchon (200) selon la revendication 2, dans lequel le manchon (202) est rempli avec au moins un élément parmi des copeaux d'os, des particules d'os, des fibres d'os, des matériaux de croissance osseuse, de l'hydroxyapathite, du métal, du matériau résorbable, du polymère, de la céramique et du ciment osseux.

16. Bouchon (200) selon la revendication 15, dans lequel le manchon (202) comprend en outre au moins un trou débouchant (208) s'étendant de la surface intérieure à la surface extérieure.

17. Bouchon (200) selon la revendication 16, dans lequel le manchon (202) est cylindrique.

18. Bouchon (200) selon la revendication 15, dans lequel le manchon (202) comprend au moins un creux s'étendant de la surface extérieure à la surface intérieure.

19. Bouchon (200) selon la revendication 2, dans lequel le manchon (202) comprend en outre une pluralité de doigts (327) formés intégralement avec celui-ci.

20. Bouchon (200) selon la revendication 2, dans lequel le manchon (202) et le premier capuchon (222) sont sensiblement formés d'os cortical.

21. Bouchon (200) selon la revendication 2, dans lequel le corps est formé d'os spongieux et le premier capuchon est formé d'os cortical.

22. Bouchon (200) selon la revendication 2, dans lequel une des extrémités supérieure et inférieure (204, 206) du corps est ouverte.

23. Bouchon (200) selon la revendication 1, comprenant en outre un second capuchon disposé sur une extrémité différente du premier capuchon.

24. Bouchon (200) selon la revendication 1, comprenant en outre :
un manchon (202) comprenant l'extrémité supérieure (204), l'extrémité inférieure (206), une surface intérieure, la surface extérieure disposée entre les extrémités supérieure et inférieure (204, 206), une rainure (216) disposée sur la surface intérieure à proximité d'une des extrémités (204, 206) et une pluralité de trous débouchants (208) s'étendant de la surface intérieure à la surface extérieure ;
le premier capuchon (222) comprenant des faces opposées (224, 226), la partie de verrouillage (228) étant configurée et dimensionnée pour être reçue dans la rainure (216) ; et
dans lequel une des faces (224, 226) du premier capuchon (222) bute contre l'une des extrémités du manchon (202).

25. Bouchon (200) selon la revendication 24, dans lequel le manchon (202) et le premier capuchon (222) sont sensiblement formés d'os cortical.

26. Bouchon (200) selon la revendication 24, dans lequel le manchon (202) est formé d'os spongieux et le premier capuchon (222) est formé d'os cortical.

27. Bouchon (200) selon la revendication 24, dans lequel une des extrémités supérieure et inférieure (204, 206) du corps est ouverte.

28. Bouchon (200) selon la revendication 24, dans lequel le manchon (202) comprend en outre un filetage (286) disposé sur la surface extérieure de celui-ci.

29. Bouchon (200) selon la revendication 24, dans lequel le manchon (202) comprend en outre une fente (214) croisant la rainure (216).

30. Bouchon (200) selon la revendication 29, dans lequel la fente (214) présente une largeur dimensionnée pour permettre le passage d'une portion de la partie de verrouillage (228) du capuchon.

31. Bouchon (200) selon la revendication 24, dans lequel la surface extérieure du manchon (202) est sensiblement cylindrique.

32. Bouchon (200) selon la revendication 24, dans lequel la surface extérieure du manchon (202) est sensiblement arquée.

33. Bouchon (200) selon la revendication 29, dans lequel le manchon (202) comprend en outre un axe central et une lèvre périphérique (236) s'étendant vers l'axe central.

34. Bouchon (200) selon la revendication 33, dans lequel la lèvre périphérique (236) est disposée à proximité d'une extrémité du manchon (202).

35. Bouchon (500) selon la revendication 24, dans lequel le manchon comprend en outre un chanfrein (511) à proximité d'une extrémité du manchon (510).

36. Bouchon (200) selon la revendication 24, dans lequel la partie de verrouillage (228) est intégralement formée avec le premier capuchon (222).

37. Bouchon (200) selon la revendication 24, dans lequel la partie de verrouillage (228) comprend un bord extérieur sensiblement arqué.

38. Bouchon (200) selon la revendication 24, dans lequel 1a partie de verrouillage (228) comprend un bord extérieur sensiblement circulaire.

39. Bouchon (200) selon la revendication 24, dans lequel la partie de verrouillage (296) comprend un bord extérieur muni d'ondulations.

40. Bouchon (200) selon la revendication 39, dans lequel les ondulations forment un bord cannelé.

41. Bouchon (200) selon la revendication 24, dans lequel la partie de verrouillage (228) est généralement symétrique autour d'une ligne centrale et s'effile depuis une partie large (232) vers une partie étroite (230).

42. Bouchon (200) selon la revendication 41, dans lequel la partie de verrouillage (228) s'effile depuis la partie large (232) vers la partie étroite (230) le long d'un rayon de courbure compris entre 0,5 cm et 1,1 cm.

43. Bouchon (200) selon la revendication 41, dans lequel les bords extérieurs des parties étroite et large (230, 232) sont disposés sensiblement le long de la circonférence d'un cercle.

44. Bouchon (200) selon la revendication 24, dans lequel la partie de verrouillage (228) est disposée sur une des faces (224, 226) du premier capuchon (222).

45. Bouchon (200) selon la revendication 24, dans lequel le premier capuchon (222) comprend en outre un premier diamètre extérieur et le manchon (202) comprend en outre un second diamètre extérieur, les premier et second diamètres extérieurs étant identiques.

46. Bouchon (320) selon la revendication 24, dans lequel le manchon comprend en outre une pluralité de doigts (327) formés intégralement avec celui-ci.

47. Bouchon (200) selon la revendication 24, comprenant en outre un second capuchon disposé sur une extrémité différente du premier capuchon (222).

48. Bouchon (200) selon la revendication 24, dans lequel le manchon (202) comprend en outre une butée pour limiter la rotation de la partie de verrouillage (228) du premier capuchon (222) quand elle est disposée dans la rainure (216).

49. Bouchon (200) selon la revendication 48, dans lequel la butée interrompt la rainure (216).

50. Bouchon (200) selon la revendication 24, dans lequel la rainure (216) s'étend de manière périphérique autour de la surface intérieure du manchon (202).

51. Bouchon (200) selon la revendication 24, comprenant en outre un insert formé d'os et configuré et dimensionné pour être reçu dans le manchon (202).

52. Bouchon (600) selon la revendication 24, dans lequel les trous débouchants (604) sont disposés dans une pluralité de rangées.

53. Bouchon (600) selon la revendication 52, dans lequel les rangées sont disposées en plans généralement parallèles.

54. Bouchon (600) selon la revendication 52, dans lequel les trous débouchants (604) sont disposés à un intervalle angulaire généralement constant les uns par rapport aux autres tel que mesuré depuis un axe central du manchon.
